# EUROPÄISCHE PATENTANMELDUNG

(11) **EP 2 745 869 A1**
(43) Veröffentlichungstag der Anmeldung: **25.06.2014**
(21) Anmeldenummer: 12199220.0
(22) Anmeldetag: 21.12.2012
(51) Int. Cl.: A61M 25/01, A61M 1/10, A61M 39/22, A61M 39/06, A61M 25/06, A61M 25/00

(54) **Schleusenanordnung für die Einführung eines strangförmigen Körpers, insbesondere eines Katheters, in einen Patientenkörper**

(71) Anmelder: ECP Entwicklungsgesellschaft mbH, 12247 Berlin (DE)
(72) Erfinder: Schumacher, Jörg, 14513 Teltow (DE); Bredenbreuker, Lars, 10781 Berlin (DE); Decke, Robert, 12247 Berlin (DE)
(74) Vertreter: Pfenning, Meinig & Partner GbR

(57) **Zusammenfassung**

Die Anmeldung betrifft eine Schleusenanordnung für die Einführung eines strangförmigen Körpers (32, 105) in einen Patientenkörper mit einer Einführschleuse (101), deren distales Ende (102) zum Einbringen in einen Patientenkörper vorgesehen ist und deren proximales Ende (103) beim Einbringen des distalen Endes in einen Patientenkörper aus diesem herausragt, und mit einer Hilfsschleuse (104) zur Einführung in die Einführschleuse (101) gemeinsam mit dem strangförmigen Körper (32, 105), mit ersten Befestigungsmitteln (106) zur Befestigung der Hilfsschleuse an der Einführschleuse, mit zweiten Befestigungsmitteln (107) zur Befestigung des strangförmigen Körpers an der Hilfsschleuse, wobei die Einführschleuse (101) eine erste Spüleinrichtung (108) zum Spülen des Schleuseninnenraums (110) aufweist, wobei die Hilfsschleuse (104) eine zweite Spüleinrichtung (109) für den zweiten Schleuseninnenraum (111) aufweist.

## Beschreibung

Die Erfindung liegt auf dem Gebiet der Mechanik und ist mit besonderem Vorteil in der Medizintechnik anwendbar. Sie bezieht sich speziell auf eine Schleusenanordnung, die es ermöglicht, einen strangförmigen Körper, insbesondere einen Katheter, wenigstens teilweise in einen Patientenkörper einzuführen.

Grundsätzlich ist es bekannt, zur Einführung eines Katheters in einen Patientenkörper, beispielsweise in ein Blutgefäß, eine Einführschleuse zu benutzen, deren proximales Ende aus dem Patientenkörper herausragt, während das distale Ende dauerhaft im Patientenkörper verbleibt. Solche Einführschleusen können beispielsweise einwachsend gestaltet sein, sie können jedoch auch einfach steril durch die Haut oder eine Körperöffnung des Patienten eingeführt werden. Eine solche Schleuse ist beispielsweise vorteilhaft, wenn ein Katheter in ein Blutgefäß eines Patienten eingeführt und aus diesem auch wieder entnommen werden soll. Eine besondere Anwendung solcher Katheter stellen Katheter mit Funktionselementen dar, beispielsweise Hohlkatheter, an deren Ende sich eine Blutpumpe befindet, die durch ein Blutgefäß bis zu einer Herzkammer oder bis zu einem größeren Blutgefäß transportiert wird, um dort gegebenenfalls expandiert zu werden. Entsprechende Blutpumpen, die expandierbar und später wieder komprimierbar sind, sind aus der Literatur vielfältig bekannt.

Als Funktionselemente können jedoch auch andere Geräte an dem Katheter befestigt sein, wie beispielsweise Fräsen zur Beseitigung von Ablagerungen in Blutgefäßen oder ähnliche.

Speziell zur Einführung von Katheterpumpen ist bereits vorgeschlagen worden, zusätzlich zu einer Einführschleuse eine Hilfsschleuse zu verwenden, in die der Katheter mit der komprimierten Pumpe eingebracht werden kann, wobei die Hilfsschleuse den endseitigen Teil des Katheters sowie die komprimierte Pumpe vorübergehend aufnimmt und gemeinsam mit der Pumpe in die Einführschleuse eingeführt wird.

Es ist bekannt, innerhalb einer Schleuse dort, wo ein Gegenstand in die Schleuse eingeführt wird, eine Spüleinrichtung innerhalb eines Schleuseninnenraums vorzusehen, um vor dem Einführen oder während des Einführens durch das Spülen des einzuführenden Körpers und/oder des Schleuseneingangs das Eindringen von Keimen beziehungsweise Luft in den Patientenkörper zu verhindern. Zudem ist es auch bekannt, an derartigen Schleusen Dichtungseinrichtungen vorzusehen, um einerseits vor dem Einführen eines Körpers durch die Schleuse das Austreten von Blut oder anderen Körperflüssigkeiten aus der Schleuse zu verhindern und andererseits während des Einführens eines Körpers in die Schleuse die Öffnung, durch die der Körper in die Schleuse eingeführt wird, so weit wie möglich gegenüber dem Außenbereich abzudichten.

Der vorliegenden Erfindung liegt vor dem geschilderten Hintergrund die Aufgabe zugrunde, eine Schleusenanordnung mit einer Einführschleuse und einer Hilfsschleuse zu schaffen, die das Einführen eines strangförmigen Körpers, insbesondere eines Katheters, mittels der Hilfsschleuse in die Einführschleuse in möglichst komfortabler Weise erlaubt und dabei insgesamt das Eindringen von Keimen oder anderen unerwünschten Gegenständen und Stoffen in den Patientenkörper möglichst weitgehend verhindert. Zudem soll die Schleusenanordnung möglichst gut gegen das Austreten von Körperflüssigkeiten geschützt sein.

Die Aufgabe wird mit den Merkmalen der Erfindung gemäß Patentanspruch 1 gelöst.

Demgemäß bezieht sich die Erfindung auf eine Schleusenanordnung für die Einführung eines strangförmigen Körpers, insbesondere eines Katheters, in einen Patientenkörper mit einer Einführschleuse, deren distales Ende zum Einbringen in einen Patientenkörper vorgesehen ist und deren proximales Ende beim Einbringen des distalen Endes in einen Patientenkörper aus diesem herausragt, und mit einer Hilfsschleuse zur Einführung in die Einführschleuse gemeinsam mit dem strangförmigen Körper / Katheter, mit ersten Befestigungsmitteln zur, insbesondere lösbaren, Befestigung der Hilfsschleuse an der Einführschleuse, mit zweiten Befestigungsmitteln zur, insbesondere lösbaren, Befestigung des strangförmigen Körpers an der Hilfsschleuse, wobei die Einführschleuse einen ersten Schleuseninnenraum mit einem ersten Aufnahmekanal für einen strangförmigen Körper sowie eine erste Spüleinrichtung zum Spülen des Schleuseninnenraums aufweist, wobei die Hilfsschleuse einen zweiten Schleuseninnenraum mit einem zweiten Aufnahmekanal für einen strangförmigen Körper sowie eine zweite Spüleinrichtung für den zweiten Schleuseninnenraum aufweist.

Bei der erfindungsgemäßen Schleusenanordnung ist sichergestellt, dass die Hilfsschleuse dauerhaft und zuverlässig an der Einführschleuse befestigt werden kann und dass so sowohl die Einführschleuse als auch die Hilfsschleuse gespült und damit sauber und keimfrei gehalten werden kann. Im Inneren der Einführschleuse kann der Innenraum zwischen der Hilfsschleuse und der Einführschleuse bzw. zwischen dem distalen Teil der Hilfsschleuse und der Einführschleuse gespült und damit keimfrei gehalten werden. Innerhalb der Hilfsschleuse kann der Innenbereich der Hilfsschleuse mit dem in dieser befindlichen oder in diese hineinragenden strangförmigen Körper, insbesondere Katheter, gespült und damit keimfrei gehalten werden. Es ergibt sich damit eine Gesamtanordnung, die dauerhaft mechanisch zusammenhält, zumindest bis die Verbindung zwischen der Einführschleuse und der Hilfsschleuse willentlich gelöst wird, und die am Übergang von der Einführschleuse zur Hilfsschleuse und am Übergang von der Hilfsschleuse zum in dieser befindlichen strangförmigen Körper in einfacher Weise eine Spülung zulässt. Damit kann sichergestellt werden, dass einerseits die Zahl der Keime und/oder anderen Verunreinigungen, die von außen in die Einführschleuse gelangen, minimiert wird und andererseits der Blutaustritt aus der Einführschleuse und der Hilfsschleuse verhindert wird.

Eine vorteilhafte Ausgestaltung der Erfindung sieht vor, dass die Hilfsschleuse einen schlauchartigen, distalen Teil und ein proximales Schleusengehäuse aufweist und dass nur der schlauchartige Teil mit der Einführschleuse, insbesondere lösbar, verbunden ist.

Dabei kann der schlauchartige Teil der Hilfsschleuse in einfacher Weise innerhalb der Einführschleuse geklemmt und damit lösbar befestigt sein. Innerhalb der Hilfsschleuse kann durch eine Spüleinrichtung die Außenseite des schlauchartigen Teils der Hilfsschleuse und das Innere der Einführschleuse mittels einer Spüleinrichtung keimfrei gehalten werden. Innerhalb des schlauchartigen Teils der Hilfsschleuse kann beim Einführen dieses Teils in die Einführschleuse bereits ein strangförmiger Körper / Katheter, beispielsweise mit einer komprimierten Blutpumpe, enthalten sein. Die Blutpumpe kann dann nach Einführen des schlauchartigen Teils der Hilfsschleuse in die Einführschleuse seinerseits von der Hilfsschleuse in die Einführschleuse, beispielsweise einen schlauchartigen Teil der Einführschleuse, überführt werden. Dabei wird gleichzeitig durch Spülen des Schleuseninnenraums der Einführschleuse die Keimfreiheit der Umgebung bei dem Transfervorgang sichergestellt.

Mit der Einführschleuse kann stirnseitig ein Sterilschlauch fluiddicht verbunden sein, in dem der schlauchartige Teil der Hilfsschleuse angeordnet sein kann. Der Sterilschlauch kann mit der Hilfsschleuse, insbesondere deren Gehäuse, fluiddicht abschließen. Der Sterilschlauch kann axial komprimierbar sein, um nach dem Einbringen des strangförmigen Körpers in die Einführschleuse die Hilfsschleuse, insbesondere deren schlauchförmigen Teil, in die Einführschleuse einzuschieben und dort zu belassen. Der Sterilschlauch kann beispielsweise als Wellschlauch ausgeführt sein.

Eine weitere vorteilhafte Ausgestaltung der Erfindung sieht vor, dass die Hilfsschleuse einen schlauchartigen, distalen Teil und ein Schleusengehäuse aufweist und dass das Schleusengehäuse der Hilfsschleuse, insbesondere lösbar, mit der Einführschleuse, insbesondere mit einem Schleusengehäuse der Einführschleuse, verbunden ist.

Wenn das Schleusengehäuse der Hilfsschleuse mit dem Schleusengehäuse der Einführschleuse fest verbunden ist, so ergibt sich insgesamt eine feste und unverformbare Einheit, die am Patientenkörper besonders einfach zu handhaben ist. Es ist dann auch besonders einfach, den strangförmigen Körper durch die Hilfsschleuse hindurch in die Einführschleuse einzuschieben. Nach der Verbindung der Hilfsschleuse mit der Einführschleuse wirkt die Schleusenanordnung wie eine einzige, mehrstufige Schleuse.

Die Erfindung kann außerdem vorteilhaft dadurch ausgestaltet werden, dass die Verbindung der Hilfsschleuse mit der Einführschleuse als lösbare Klemmverbindung mit Kraft- oder Reibschluss, als Doppelkonusverbindung, Schraubverbindung, Bajonettverbindung, Presspassung oder andere lösbare Passverbindung ausgeführt ist. Derartige lösbare Verbindungen sind auch unter Operationsbedingungen einfach herstellbar und wieder lösbar. Wegen der einfachen mechanischen Funktion werden keine chemisch aktiven Fügemittel benötigt, und es gibt somit keine Kontaminationsrisiken bei der Verwendung am lebenden Patienten. Die Lösung der entsprechenden Verbindung ist ebenso einfach wie die Herstellung der Verbindung.

Einzelne Beispiele für entsprechende lösbare Verbindungen werden weiter unten in Verbindung mit den Ausführungsbeispielen näher erläutert.

Die Erfindung kann außerdem vorteilhaft dadurch realisiert werden, dass die zweite Spüleinrichtung mit der ersten Spüleinrichtung verbunden ist. Dabei ist insbesondere vorgesehen, dass ein Fluidaustausch zwischen der ersten und der zweiten Spüleinrichtung stattfinden kann, so dass für die beiden Spüleinrichtungen dasselbe Fluid verwendet wird. Dies senkt den Aufwand und erhöht die Sicherheit der Keimfreiheit. Es kann beispielsweise auch für die erste und die zweite Spüleinrichtung dieselbe Pumpe zur Bewegung des Spülfluids verwendet werden.

Es kann dabei vorteilhaft vorgesehen sein, dass der Innenraum des Schleusengehäuses der Hilfsschleuse mittels eines Hilfsspülkanals unmittelbar mit der ersten Spüleinrichtung verbunden ist. Dabei kann als Implementierung beispielsweise vorgesehen sein, dass der Hilfsspülkanal durch eine umfangsseitig außen am Gehäuse der Hilfsschleuse angeschlossene Leitung in Form eines Schlauchs oder Rohrs verläuft. Damit kann die Spüleinrichtung der Hilfsschleuse ebenso wie die Spüleinrichtung der Einführschleuse aufgebaut sein, wobei lediglich die beiden Spülkanäle miteinander verbunden sind.

Es kann aber auch vorteilhaft vorgesehen sein, dass der Hilfsspülkanal unmittelbar vom Schleusengehäuse der Einführschleuse in das Innere des schlauchartigen Teils der Hilfsschleuse verläuft, insbesondere durch mantelseitige Öffnungen in dem schlauchartigen Teil der Hilfsschleuse. Somit kann innerhalb des Innenraums des Schleusengehäuses der Einführschleuse außer dem Außenbereich der in der Einführschleuse befindlichen Hilfsschleuse auch das Innere der Hilfsschleuse, insbesondere das Innere eines schlauchförmigen Teils der Hilfsschleuse, mitgespült werden, indem der schlauchartige Teil der Hilfsschleuse mantelseitige Öffnungen aufweist, durch die das Spülfluid eindringen kann. Das Spülfluid kann vorteilhaft sodann in axialer Richtung entlang des schlauchartigen Teils der Hilfsschleuse fließen, um die Hilfsschleuse insgesamt zu spülen.

Dabei kann das Spülfluid von der Einführschleuse aus bis in das Schleusengehäuse der Hilfsschleuse gelangen und sich dabei grundsätzlich zwischen der Hilfsschleuse und dem in dieser befindlichen strangförmigen Körper /Katheter bewegen, so dass auch der Katheter / strangförmige Körper als solcher keimfrei gehalten wird. Dieser wird in den Patientenkörper eingeschoben, so dass gerade der strangförmige Körper selbst / der Katheter keimfrei gehalten werden muss.

Eine weitere vorteilhafte Ausgestaltung der Erfindung sieht vor, dass im Fall einer unmittelbaren Verbindung des Schleusengehäuses der Hilfsschleuse mit dem Schleusengehäuse der Einfuhrschleuse der Hilfsspülkanal innerhalb des Verbindungsbereichs, insbesondere durch einen innerhalb der Einführschleuse angeordneten Bereich der Wand des Schleusengehäuses der Hilfsschleuse hindurch oder auch durch einen von einem Teil der Hilfsschleuse umgebenen Teil der Wand des Gehäuses der Einführschleuse, verläuft. Bei dieser Ausgestaltung wird eine feste, kanalartige Verbindung zwischen dem Innenraum der Einführschleuse und dem Innenraum des Gehäuses der Hilfsschleuse hergestellt, die zuverlässig den Übergang des Spülfluids zwischen den Innenräumen der Gehäuse der beiden Schleusen erlaubt. Es kann zudem wenigstens eine mantelseitige Öffnung am Gehäuse der Hilfsschleuse vorgesehen sein, die entweder dem Abfließen des Spülfluids oder der Entlüftung dienen kann.

Beispielsweise kann alternativ hierzu oder zusätzlich auch am distalen Ende der Hilfsschleuse eine Hohlnadel angebracht sein, die beim Einschieben der Hilfsschleuse in die Einführschleuse eine Gummidichtung der Einführschleuse, beispielsweise eine Dom- oder Plattendichtung, durchstößt und bis in das Innere des Spülraums der Einführschleuse hineinragt. Damit wird durch den Hohlraum der Nadel ein Kommunikationskanal zwischen dem Innenraum des Gehäuses der Einführschleuse und dem Gehäuse der Hilfsschleuse geschaffen.

Eine vorteilhafte Ausgestaltung der Erfindung sieht außerdem vor, dass die Einführschleuse und die Hilfsschleuse in einem ringförmigen Bereich als Verbindungspartner aneinander anliegen und dass in dem ringförmigen Bereich eine ringförmig oder ringsegmentförmig in Umfangsrichtung umlaufende Ringnut in wenigstens einem der beiden Verbindungspartner vorgesehen ist und dass sowohl in dem Schleusengehäuse der Einführschleuse als in dem Schleusengehäuse der Hilfsschleuse jeweils eine kanalartige Ausnehmung vorgesehen ist, die den Schleuseninnenraum der jeweiligen Schleuse im verbundenen Zustand mit der Ringnut verbindet. Dabei kann entweder ein Gehäuseteil der Einführschleuse im Verbindungsbereich ein Gehäuseteil der Hilfsschleuse umgeben, oder es kann umgekehrt ein Gehäuseteil der Hilfsschleuse ein Gehäuseteil der Einführschleuse umgeben.

Dadurch, dass die kanalartigen Ausnehmungen im Gehäuse, insbesondere der Gehäusewand, der Einführschleuse und im Gehäuse, insbesondere der Gehäusewand, der Hilfsschleuse mittels eines ringförmigen oder ringsegmentförmigen Kanals miteinander verbunden werden, kann auch eine Verdrehung der Hilfsschleuse gegenüber der Einführschleuse die Herstellung der Verbindung zwischen den kanalartigen Ausnehmungen nicht verhindern. Dies schafft eine große Zuverlässigkeit bei der Herstellung der Verbindung der Spülräume der Einführschleuse und der Hilfsschleuse, sobald die beiden Gehäuse miteinander verbunden sind, unabhängig von einer möglicherweise eintretenden Verdrehung der Teile gegeneinander. Die kanalartigen Ausnehmungen bzw. der Ringkanal können dabei derart gestaltet werden, dass zumindest der im Gehäuse der Einführschleuse vorgesehene Kanal, insbesondere auch beide Kanäle, durch einen Schieber geschlossen wird, sobald die beiden Schleusengehäuse voneinander getrennt werden.

Im Folgenden wird die Erfindung anhand eines Ausführungsbeispiels in einer Zeichnung gezeigt und nachfolgend beschrieben. Dabei werden zunächst mögliche Befestigungsarten einer Hilfsschleuse in einer Einführschleuse bzw. eines Katheters in einer Hilfsschleuse beschrieben, bevor dann auf die Kombination der Spüleinrichtungen und die Befestigung der beiden Schleusen aneinander näher eingegangen wird.
- Fig. 1: eine schematische Übersicht über ein Gefäßsystem mit einer eingeführten Schleuse;
- Fig. 2: eine Detailansicht eines Ausschnitts der Fig. 1;
- Fig. 3: eine Ausführungsform der Erfindung mit einer ersten Schleuse (Einführschleuse) und einer zweiten Schleuse (Hilfsschleuse);
- Fig. 4: eine Ausführungsform einer Pumpe;
- Fig. 5: eine zweite Schleuse (Hilfsschleuse) mit einer aus dieser extrahierten Pumpe;
- Fign. 6, 7: das Einziehen einer Pumpe in eine zweite Schleuse (Hilfsschleuse);
- Fign. 8, 9: das Überführen einer Pumpe von einer zweiten in eine erste Schleuse (Einführschleuse);
- Fig. 10: einen Längsschnitt durch ein Schleusengehäuse mit einem schlauchförmigen Abschnitt;
- Fig. 11: einen Längsschnitt durch einen Teil eines Schleusengehäuses mit einer Schneidvorrichtung;
- Fig. 12: einen Längsschnitt durch ein Schleusengehäuse mit einer Klemmvorrichtung für den schlauchförmigen Abschnitt und einer weiteren Klemmvorrichtung;
- Fig. 13a: einen Längsschnitt durch einen alternativen Klemmring mit einem konischen Druckstück;
- Fig. 13b: einen Längsschnitt durch eine Klemmverbindung mit jeweils mantelseitigen Rillen und Wülsten;
- Fig. 13c: einen Längsschnitt mit einer Klemmverbindung in Form einer Gewindeverbindung;
- Fig. 13d: einen Längsschnitt mit einer Klemmverbindung in Form einer Konusverbindung mit Bajonett-Verriegelung;
- Fig. 13e: eine Ansicht des Konus aus Fig. 13d um 90° gedreht;
- Fig. 13f: im Längsschnitt eine Luer-Konusverbindung mit einer Gewindeverbindung;
- Fig. 13g: im Längsschnitt eine Klemmverbindung mit einer im Schleusengehäuse geführten, quer verschiebbaren, radial aufweitbaren Federklammer;
- Fig. 13h: eine mögliche Gestaltung des zu klemmenden Körpers im Bereich der Klemmstelle;
- Fig. 13i: eine Federklammer aus Fig. 13g in einer Seitenansicht, gegenüber Fig. 13g um 90° gedreht;
- Fig. 14: einen Längsschnitt durch ein Schleusengehäuse mit einer Klemmvorrichtung für einen proximal eingeschobenen strangförmigen Körper;
- Fig. 15: eine Einführschleuse und eine mit dieser gekoppelte Hilfsschleuse, wobei die Spüleinrichtungen beider Schleusen gekoppelt sind;
- Fig. 16: eine Einführschleuse und eine mit dieser gekoppelte Hilfsschleuse, wobei im Schleuseninnenraum der Einführschleuse eine Verbindung zum Inneren der Hilfsschleuse besteht;
- Fig. 17: eine Schleusenanordnung, bei der die Schleusengehäuse von Hilfs- und Einführschleuse fest verbunden sind; sowie
- Fig. 18: eine Einführschleuse mit einer Hilfsschleuse, die in einen Sterilschlauch aufgenommen werden kann.

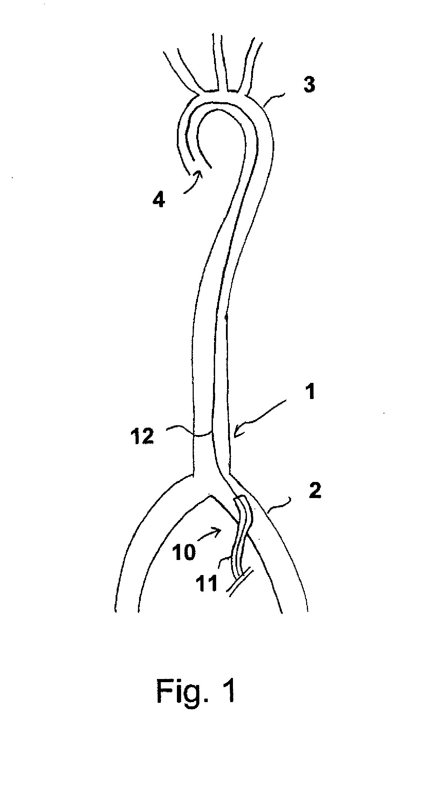

In der Figur 1 ist ein schematisches menschliches Gefäßsystem 1 gezeigt. Im Bereich der Leiste liegt eine der femoralen Arterien 2, die über eine Hauptschlagader mit dem Aortenbogen 3 verbunden ist und anschließend in die Herzkammer 4 mündet. Mithilfe beispielsweise der Seldinger-Technik wird zunächst eine Einführschleuse 10 in die femorale Arterie 2 eingeführt. Hierbei wird zunächst die femorale Arterie bzw. ein beliebiges Blutgefäß beispielsweise mit einer Stahlkanüle mit schneidender Spitze punktiert. Durch die in die Punktur eingeführte Stahlkanüle hindurch wird ein Führungsdraht 12 geschoben und retrograd über den Aortenbogen 3 in den linken Herzventrikel 4 eingeführt. Nach Entfernung der Punktionskanüle wird die als Einführschleuse ausgebildete erste Schleuse 10, welche einen schlauchförmigen Abschnitt 11 sowie gegebenenfalls einen hier nicht dargestellten Dilatator umfasst, auf den Führungsdraht eingefädelt und durch die punktierte Stelle in das Gefäßsystem eingeführt, wobei die Schleuse eine geringe Distanz in das Lumen des Gefäßsystems oder auch bis zum Einsatzort eines einzuführenden Elementes eingeführt wird. Anschließend wird eine Fluidpumpe durch die Einführschleuse 10 in das Gefäßsystem eingeführt.

Der schlauchförmige Abschnitt 11 der ersten Schleuse 10 ist derart in die Arterie eingeführt, dass das proximale Ende der ersten Schleuse 10 außerhalb der femoralen Arterie liegt und aus dem Körper des Patienten herausragt und somit zum Einführen beispielsweise einer Blutpumpe genutzt werden kann. So ist es möglich, die Pumpe auf dem Führungsdraht 12 aufzufädeln, um die Pumpe mithilfe des Führungsdrahtes bis in den linken Herzventrikel zu führen.

Es ist auch möglich, den schlauchförmigen Teil/Abschnitt 11 der ersten Schleuse / Einführschleuse 10 durch den Führungsdraht geführt bis in den linken Herzventrikel zu führen und den Führungsdraht 12 anschließend aus der ersten Schleuse zu entfernen. Eine etwaige Pumpeneinheit wird anschließend durch das erste Schleusenlumen bis in die Nähe oder bis in den linken Ventrikel 4 geführt.

Vorliegend wird das Verfahren lediglich anhand des Einführens einer Pumpe in den linken Herzventrikel zum Unterstützen einer Herzfunktion dargestellt. Für den Fachmann ist jedoch leicht erkennbar, dass die Pumpe oder ein anderes Funktionselement auch an andere Stellen im körpereigenen Gefäßsystem angeordnet und eingebracht werden kann.

Es versteht sich von selbst, dass anstelle einer Blutpumpe/Herzunterstützungspumpe auch ein anderes Funktionselement, insbesondere ein komprimierbares und expandierbares Element, mit einem Katheter über die Schleuse eingebracht werden kann.

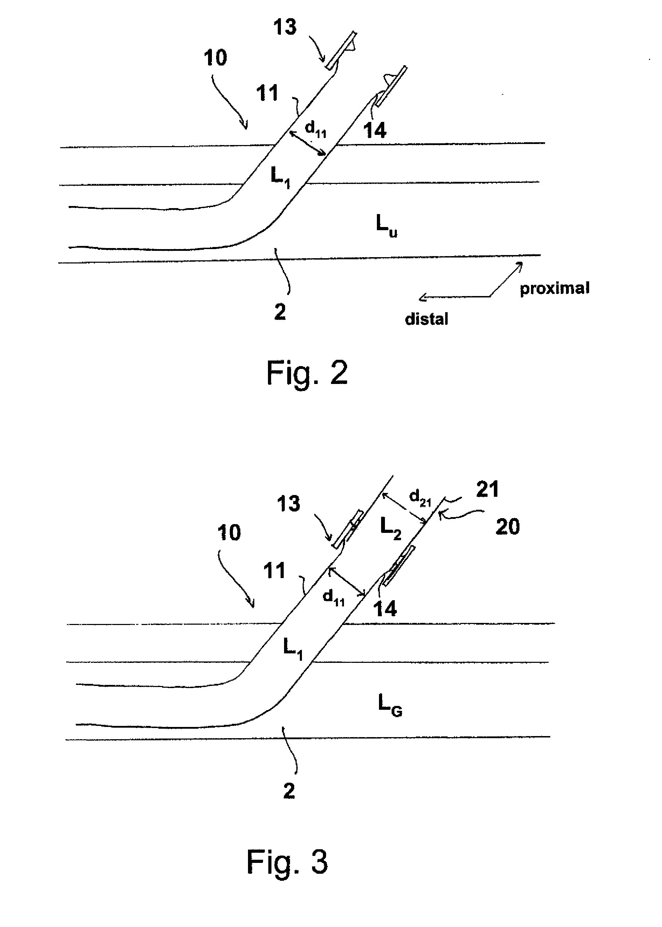

In der Figur 2 ist der Bereich der Fig. 1 dargestellt, in welchem die erste Schleuse 10 durch das körpereigene Gewebe von außen in das Lumen L_{G} der femoralen Arterie 2 geführt ist. Die erste Schleuse umfasst dabei einen schlauchförmigen Abschnitt 11, welcher proximal mit einem Schleusengehäuse 13 verbunden ist. Der schlauchförmige Abschnitt 11 definiert ein Lumen L₁, welches einen Innendurchmesser d₁₁ aufweist. Zum proximalen Ende des schlauchförmigen Abschnitts 11 hin weitet sich dieser trompetenartig im Bereich 14 auf.

Das Schleusengehäuse 13 enthält ein im Stand der Technik bekanntes hämostatisches Ventil. Dieses verhindert, dass im Lumen L_{G} befindliches Fluid durch das Lumen L₁ nach außen austreten kann.

In der Darstellung der Figur 3 ist die erste Schleuse 10 der Fig. 2 mit einer zweiten Schleuse 20 gekoppelt. Von der zweiten Schleuse 20 (Hilfsschleuse) ist lediglich ein schlauchförmiger Abschnitt 21 gezeigt, welcher ein Lumen L₂ mit einem Innendurchmesser d₂₁ definiert. Das distale Ende der zweiten Schleuse 20 weist dabei einen derartigen Außendurchmesser auf, dass es in das Schleusengehäuse 13 eingeführt werden kann. Der Innendurchmesser d₂₁ ist jedoch größer als der Innendurchmesser d₁₁.

Eine nicht dargestellte, im Lumen L₂ befindliche Pumpe kann nun durch Drücken vom zweiten Schleusenlumen L₂ in das erste Schleusenlumen L₁ überführt werden. Anschließend wird die Pumpe durch das erste Schleusenlumen L₁ bis an die Stelle im Gefäßsystem transportiert, an welcher die Pumpe ihre Wirkung entfalten soll. Hierbei kann die Pumpe entweder auf einem Führungsdraht geführt werden oder ohne Führungsdraht durch das erste Schleusenlumen eingebracht werden. Die erste Schleuse kann, um die Pumpe und die Gefäßwände sowie den Wellenkatheter zu schonen, distal bis zum Einsatzort der Pumpe vorgeschoben werden, bevor die Pumpe herausgeschoben wird.

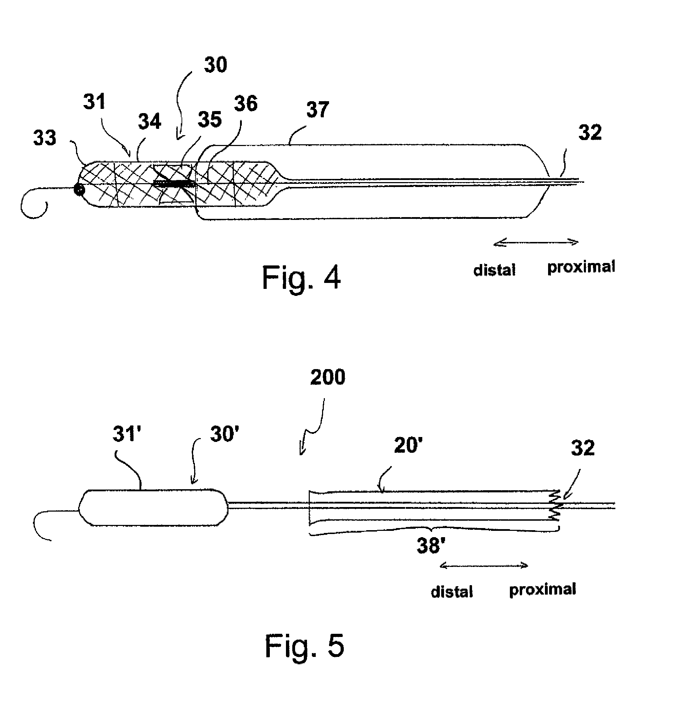

Anhand der Figur 4 wird eine mögliche Ausführung einer Pumpe 30 genauer erläutert. Die Pumpe 30 umfasst eine distale Pumpeneinheit 31 und einen sich an das proximale Ende der distalen Pumpeneinheit 31 anschließenden Wellenkatheter 32. Der Wellenkatheter 32 weist an seinem proximalen, nicht dargestellten Ende eine Kupplung zur Ankopplung des Wellenkatheters 32 an eine Antriebsvorrichtung auf. Die Antriebsvorrichtung kann außerhalb des Patientenkörpers angeordnet sein und versetzt eine im Wellenkatheter 32 verlaufende flexible Welle in Rotation, welche wiederum die distale Pumpeneinheit 31 antreibt. Die vorliegende Erfindung ist allerdings auch zur Einführung anders angetriebener Pumpen (mittels eines an der Pumpe angeordneten Elektromotors oder einer in der Pumpe angeordneten pneumatischen Turbine) geeignet.

Die distale Pumpeneinheit umfasst ein Pumpengehäuse 33, welches aus sich kreuzenden Nitinolstreben hergestellt ist. Das Nitinolgehäuse ist zu Teilen mit einer Beschichtung 34 versehen, welche sich distal und proximal eines im Gehäuse 33 angeordneten Rotors 35 erstreckt. Der Rotor ist mit der durch den Wellenkatheter 32 verlaufenden Welle 36 verbunden und wird so in Drehung versetzt. Das Gehäuse und der Rotor sind komprimierbar, d. h., die Pumpe ist eine selbstentkomprimierbare Pumpe. Die Entfaltung der Pumpe vollzieht sich nach dem Herausschieben der distalen Pumpeneinheit aus dem distalen Ende einer Schleuse. Zum Komprimieren der Pumpe in Vorbereitung der Implantation wird die distale Pumpeneinheit in das distale Ende eines Schleusenlumens einer zweiten Schleuse (Hilfsschleuse) eingezogen. Das Schleusenlumen besitzt dabei einen Innendurchmesser, welcher größer als der Außendurchmesser des Wellenkatheters sein kann.

Der Rotor kann gegenüber dem Pumpengehäuse in Axialrichtung verschiebbar sein, insbesondere mittels einer Axialverschiebung der Antriebswelle. Der Rotor kann andererseits auch in Axialrichtung gegenüber dem Pumpengehäuse fixiert sein.

Optional weist die Pumpe einen Abströmschlauch 37 auf, welcher einen proximal des Rotors 35 gelegenen Strömungskanal für das gepumpte Fluid definiert. Am proximalen Ende des Abströmschlauchs 37 befinden sich nicht näher dargestellte Auslassöffnungen.

Selbstverständlich kann die Pumpe auch von einem Pumpbetrieb in einen Saugbetrieb umgeschaltet werden, so dass die Pumpe nicht länger Fluid vom distalen Ende ans proximale Ende führt, sondern umgekehrt.

Eine detailliertere Beschreibung einer weiteren geeigneten Pumpe kann beispielsweise der Druckschrift EP 2 047 872 A1 entnommen werden. Anhand der Figuren 5 bis 9 soll nun die Funktion des Systems erläutert werden.

In der Figur 5 ist eine Pumpe 30' dargestellt, welche im Wesentlichen der Pumpe 30 nach der Fig. 4 entspricht. Zur Vereinfachung sind Details der Pumpe nicht gezeigt. Es sind lediglich das bauchige Gehäuse sowie das distal des bauchigen Gehäuses gelegene "Pigtail" dargestellt, welches ein Ansaugen der Herzpumpe an die Herzwand verhindert. Proximal der distalen Pumpeneinheit 31' verläuft der Wellenkatheter 32'. Einen Bereich 38' des Wellenkatheters 32' umschließend ist eine zweite Schleuse 20' angeordnet, welche ein Lumen L₂ umfasst, dessen Innendurchmesser d₂₁ kleiner als der Durchmesser der distalen Pumpeneinheit 31' im entfalteten Zustand ist.

Die in der Fig. 5 dargestellte Pumpe 30' ist eine komprimierbare Pumpe, das heißt, die distale Pumpeneinheit 31', welche unter anderem das Pumpengehäuse und den darin befindlichen Rotor umfasst, ist derart ausgebildet, dass sie komprimiert, d. h. in ihrem Durchmesser verringert werden kann. Nachdem ein Qualitätsprüfer oder beispielsweise ein Arzt sich von der korrekten Funktion der Pumpe 30' überzeugen konnte, z. B. durch Betrachtung der Rotationsbewegung der in der distalen Pumpeneinheit 31' befindlichen Rotoreinheit bei einem Testlauf, wird durch Ziehen des Wellenkatheters 32' in proximaler Richtung die distale Pumpeneinheit 31' in das Lumen L₂ der zweiten Schleuse 20' gezogen. Durch das Einziehen der Pumpe in die zweite Schleuse 20' wird ein Verbiegen bzw. eine Beschädigung des Wellenkatheters bzw. der darin verlaufenden Welle vermieden. Die in der Fig. 5 dargestellte Pumpe 30' und die den Bereich 38' des Wellenkatheters 32' umschließende zweite Schleuse 20' bilden ein System 200, welches es ermöglicht, rechtzeitig vor einer Operation die Funktion der Pumpe 30' zu testen und anschließend durch das Ziehen der distalen Pumpeneinheit 31' in das distale Ende der zweiten Schleuse 20' die Pumpe zu komprimieren und dabei eine Beschädigung der Welle zu vermeiden.

Obgleich das System sowohl mit aktiv entkomprimierbaren Pumpen als auch mit selbstentkomprimierbaren Pumpen realisierbar ist, eignet es sich insbesondere für selbstentkomprimierbare Pumpen, d. h. Pumpen, deren distale Pumpeneinheit außerhalb der Schleuse selbsttätig wieder die ursprüngliche Größe annimmt.

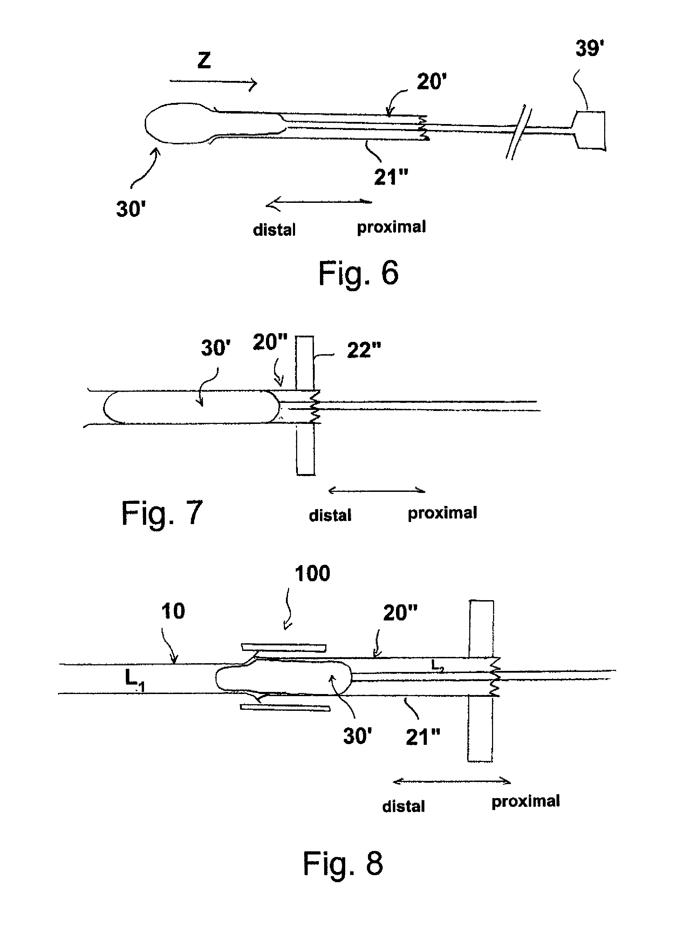

In der Figur 6 ist ein Zwischenschritt beim Einziehen der distalen Pumpeneinheit 30' in das Lumen der zweiten Schleuse 20' dargestellt. Es ist erkennbar, dass die distale Pumpeneinheit 30' komprimierbar ist und auf einen geringeren Durchmesser gebracht werden kann, so dass die distale Pumpeneinheit 30' in das Lumen der zweiten Schleuse 20' aufgenommen werden kann.

Ferner ist in der Fig. 6 eine an den Wellenkatheter 32' anschließende Kupplung 39' dargestellt, welche ein Ankoppeln der in dem Wellenkatheter verlaufenden Welle an eine Antriebseinheit ermöglicht. Da die Kupplung 39' einen oftmals größeren Außendurchmesser als den Innendurchmesser des Lumens L₂ aufweist, wird die zweite Schleuse 20' zumeist vor der Montage der Kupplung 39' vom proximalen Ende des Wellenkatheters 32' her in distaler Richtung aufgesetzt, so dass die Pumpe im System 200, d. h. die Pumpe mit der sich proximal der distalen Pumpeneinheit 31' befindlichen zweiten Schleuse 20' und der vormontierten Kupplung 39', ausgeliefert wird. An der Fig. 6 ist auch eine leichte Aufweitung des distalen Endes der zweiten Schleuse 20' dargestellt. Die trompetenartige Aufweitung 24' erleichtert das Einbringen der distalen Pumpeneinheit 31' in das Lumen L₂ der zweiten Schleuse 20',

In der Figur 7 schließlich befindet sich die distale Pumpeneinheit 31' vollständig im Lumen L₂ der zweiten Schleuse 20". Die zweite Schleuse 20" weist zwei vormontierte Griffeinheiten 22" auf, welche ein besseres Halten bzw. Entfernen der zweiten Schleuse 20" beim Einziehen der distalen Pumpeneinheit 31' in das Lumen L₂ bzw. ein anschließendes Aufreißen ermöglichen. Gemäß der Erfindung kann jedoch vorteilhaft auch eine Hilfsschleuse verwendet werden, die dauerhaft mit der Einführschleuse verbunden oder in dieser aufbewahrt bleibt. Vorteilhafterweise wird bei vorhandenem "Pigtail" dieses ebenfalls in das Lumen L₂ eingezogen, so dass sich die distale Pumpeneinheit 31' samt distal der distalen Pumpeneinheit 31' gelegenen Komponenten der Pumpe im Lumen L₂ befindet.

In der Figur 8 wird erkennbar, wie das System 200 aus Pumpe 30' und zweiter Schleuse 20" in Wirkverbindung mit der ersten Schleuse 10 zu einem System 100 kombiniert wird. Zunächst wird die zweite Schleuse 20" (Hilfsschleuse) mit ihrem distalen Ende in das Schleusengehäuse der ersten Schleuse 10 (Einführschleuse) eingeführt. Sobald die distale Spitze der zweiten Schleuse 20" an der Mündung des schlauchförmigen Abschnitts der ersten Schleuse 10 anliegt, wird durch Schieben der Pumpe in distaler Richtung, wobei das Schieben anhand eines Schiebens des Wellenkatheters 32' erfolgt, die Pumpe von der zweiten Schleuse 20' in die erste Schleuse 10' überführt. Hierbei wird der Durchmesser der distalen Pumpeneinheit 31' weiter auf den Innendurchmesser d₁₁ des Lumens L₁ reduziert.

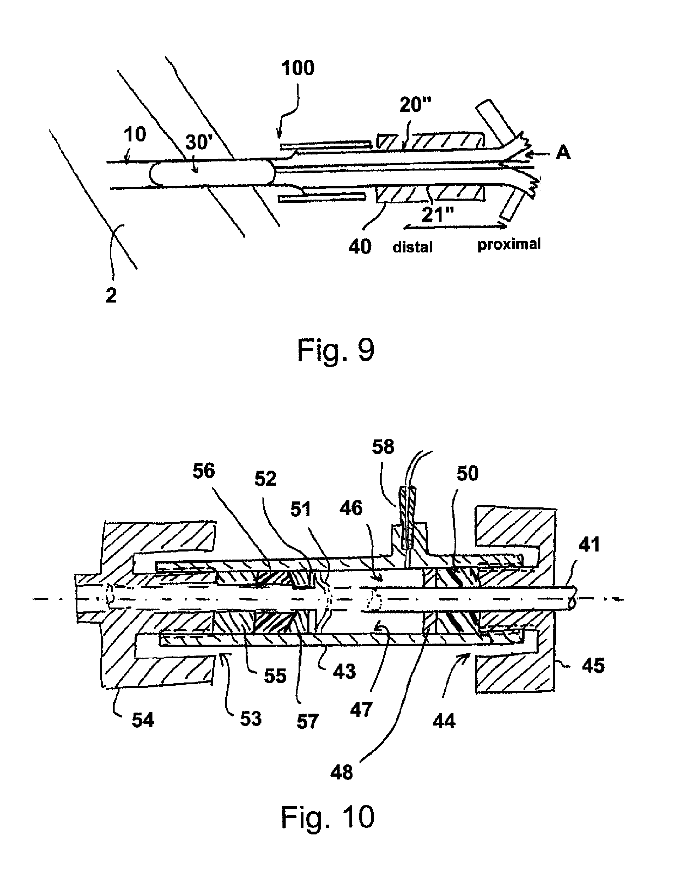

In der Figur 9 ist der anschließende Schritt dargestellt, bei welchem sich die distale Pumpeneinheit 31' vollständig im Lumen L₁ der ersten Schleuse 10 befindet. Dass sich die distale Pumpeneinheit 31' vollständig im Lumen L₁ der ersten Schleuse 10 befindet, kann beispielsweise anhand einer farblichen Markierung 50, welche auf der Außenseite des Wellenkatheters 32' aufgebracht ist, kenntlich gemacht werden.

Anschließend kann gegebenenfalls die zweite Schleuse 20", sofern sie als eine "Peel-away"-Schleuse ausgebildet ist, vom Wellenkatheter 32' entfernt werden, indem die Peel-away-Schleuse vom proximalen zum distalen Ende hin aufgerissen und vom Wellenkatheter 32' abgezogen wird. Das gerichtete Aufreißen vom proximalen zum distalen Ende hin kann durch Einkerbungen A unterstützt werden, beruht jedoch vorwiegend auf der Ausrichtung der Molekülketten des verwendeten Kunststoffs von der proximalen in die distale Richtung.

Nachdem gegebenenfalls die Schleuse entfernt wurde, wird die Pumpe 30' weiter innerhalb des Lumens L₁ der ersten Schleuse 10 bis zum gewünschten Ort geführt.

Wahlweise kann die erste Schleuse auch vor oder nach dem Einführen der Pumpe mit der distalen Schleusenmündung in die unmittelbare Nähe des Einsatzortes vorgeschoben werden. Hierzu weist die erste Schleuse die notwendige Länge auf.

Eine Versteifung der zweiten Schleuse 20" ist insbesondere beim Einziehen der distalen Pumpeneinheit 31' in das distale Ende des zweiten Schleusenlumens L₂ nicht notwendig, da die Gefahr eines Abknickens der Welle bei einer Ziehbewegung stark reduziert ist.

Beim Überführen der Pumpe von der zweiten Schleuse in die erste Schleuse, wie anhand der Figuren 7 bis 9 dargestellt, kann die zweite Schleuse eine sie verstärkende Struktur in Form eines eingebrachten Drahtes umfassen, oder der schlauchförmige Abschnitt 21" der Schleuse 20" wird nicht aus einem flexiblen Kunststoff, sondern aus einem formfesten Kunststoff oder Metall hergestellt.

Eine weitere Möglichkeit zum Stabilisieren der Pumpe und der zweiten Schleuse besteht darin, beim Vorschieben der Pumpe 30' in distaler Richtung, d. h. insbesondere beim Überführen der Pumpe 30' von der zweiten Schleuse in die erste Schleuse, die zweite Schleuse 20" durch eine Stützvorrichtung 40 in Form einer stabilen äußeren Hülse und/oder in einem Aufnahmekanal der Einführschleuse zu halten.

Anschließend soll noch eine mögliche Variante eines Verfahrens zum Einführen einer Pumpe in einen linken Herzventrikel geschildert werden. Als vorbereitende Maßnahme wird die Pumpe zunächst mit steriler physiologischer Kochsalzlösung befüllt und somit komplett entlüftet. Anschließend wird die proximal der distalen Pumpeneinheit gelegene Hilfsschleuse bis zu einem eventuell vorhandenen Abströmschlauch vorgeschoben. Die Hilfsschleuse besitzt einen Durchmesser von beispielsweise 10 Fr. Nachdem die Hilfsschleuse bis zum Abströmschlauch vorgeschoben wurde, wird die Hilfsschleuse von einer hülsenförmigen, stützenden Vorrichtung umfasst. Anschließend wird die distale Pumpeneinheit, gegebenenfalls unter leichter Drehbewegung, in die Hilfsschleuse eingezogen, indem am Wellenkatheter eine Zugbewegung in proximaler Richtung ausgeübt wird. Die Pumpe wird so weit in die Hilfsschleuse verschoben, dass ein eventuell vorhandenes Pigtail ebenfalls in der Hilfsschleuse geborgen ist. Durch diese Schritte ist es möglich, die Funktionsfähigkeit der Pumpe bereits vor einem operativen Eingriff zu überprüfen und die Pumpe erst anschließend in eine Schleuse einzuführen, ohne unter Zeitdruck agieren zu müssen. Zum Beispiel erst anschließend wird die Punktur des Gefäßsystems zum Einführen der ersten Schleuse durchgeführt. Zur Zeiteinsparung ist es auf diese Weise aber auch möglich, dass ein Assistent die Pumpe vorbereitet während der Anwender bereits parallel die Punktion durchführt.

Nachdem beispielsweise eine 9Fr-Einführschleuse bis in den linken Herzventrikel eingeführt wurde, wird ein eventuell vorhandener Dilatator aus der Einführschleuse gezogen und aus dieser entfernt.

Anschließend wird die in der Hilfsschleuse gehaltene Pumpe, welche beispielsweise anfangs von der Hülse umfasst wird, in das Schleusengehäuse der Einführschleuse eingeschoben, bis die Spitze der Hilfsschleuse an einem mechanischen Anschlag anschlägt. Anschließend wird die Pumpe durch Schieben des Wellenkatheters von der Hilfsschleuse in den schlauchförmigen Abschnitt der Einführschleuse überführt. Sobald die distale Pumpeneinheit komplett in die Einführschleuse überführt wurde, wie dies beispielsweise anhand einer optischen Markierung auf dem Wellenkatheterschaft überprüfbar ist, kann die Hilfsschleuse in der Einführschleuse belassen oder gegebenenfalls eine Peel-away-Schleuse aufgerissen und vom Wellenkatheter abgezogen werden. Anschließend wird die Pumpe innerhalb der ersten Schleuse (Einführschleuse) bis in den linken Herzventrikel vorgeschoben. Die erste Schleuse wird anschließend aus dem linken Herzventrikel bis zum Beginn der absteigenden Aorta zurückgezogen.

Die Positionierung der distalen Pumpeneinheit im linken Herzventrikel kann beispielsweise durch Röntgendurchleuchtung kontrolliert werden. Hierzu befindet sich eine röntgensichtbare Markierung am Pumpengehäuse bzw. in dessen Nähe, beispielsweise am Katheter, oder das Pumpengehäuse selbst ist röntgensichtbar. Ebenso sollte der Auslassbereich der Pumpe, d. h. die Ausströmöffnungen eines Abströmschlauches, im Bereich der aufsteigenden Aorta liegen. Auch dies kann mit einer röntgensichtbaren Markierung überprüft werden. Eine eventuell vorhandene Pigtail-Katheterspitze sollte an der Spitze des linken Herzventrikels anstoßen.

Um die Pumpe aus dem Herzventrikel zu entfernen, wird diese mittels am Wellenkatheter aufgebrachter Zugkraft in die Einführschleuse zurückgezogen und komprimiert aus dem arteriellen Gefäßsystem entfernt. Sie kann auch, wenn die erste Schleuse bereits gekürzt ist, zunächst in den Wellenkatheter ein Stück weit zurückgezogen werden, um die Pumpe zu komprimieren. Hierzu kann der Wellenkatheter einen Einzugtrichter aufweisen, in den die Pumpe durch Zug an der Antriebswelle eingezogen werden kann. Anschließend werden die erste Schleuse und weitere verbliebene Komponenten aus dem Gefäßsystem entfernt.

Besonderen Vorteil bringt bei der Erfindung die Verwendung einer langen Schleuse insbesondere als Einführschleuse bei der Implantation und Explantation der Pumpe. Die lange Schleuse dient nicht nur, wie im Stand der Technik üblich, zum Einführen der Pumpe in ein körpereigenes Lumen, sondern zur Führung der Pumpe durch das Schleusenlumen in die Nähe des Wirkungsorts. Hierbei ist es vorteilhaft, wenn im medizinischen Bereich die Schleuse eine Länge zwischen 40 und 120 cm besitzt. Die Länge wird durch den späteren Wirkungsort der Pumpe und die Physis des Patienten bestimmt.

Wird die Pumpe zusammen mit der langen Schleuse aus einem körpereigenen Lumen herausgezogen, so wird die Blutung der femoralen Arterie mit einem Druckverband gestillt. Alternativ kann die Pumpe aus dem Schleusenlumen der langen Schleuse herausgezogen werden. Dann kann durch das Lumen der Schleuse ein weiterer Führungsdraht platziert werden, über den dann nach Entfernung der Schleuse eine Vorrichtung zum Schließen der Punktur geführt werden kann. Hierdurch ist eine verbesserte Blutungsstillung erreichbar.

Die Figuren 10 bis 13 zeigen speziell eine erfindungsgemäße Ausführung der ersten Schleuse mit einer bzw. mehreren Klemmvorrichtungen zur Fixierung eines schlauchförmigen Abschnittes 41 in einem Schleusengehäuse 43.

Figur 10 zeigt dazu in einem Längsschnitt ein Schleusengehäuse 43, das im Wesentlichen die Form einer zylindrischen Hülse aufweist, die zumindest an dem distalen, dem Patientenkörper zugewandten Ende 44 durch eine Druckschraube 45 abgeschlossen ist. Das Schleusengehäuse 43 weist einen durchgehenden Aufnahmekanal 46 für einen schlauchförmigen Abschnitt 41 der Schleuse auf. In der Darstellung der Fig. 10 ist der schlauchförmige Abschnitt 41 bis in den Spülraum 47 des Aufnahmekanals 46 durchgehend, anschließend in proximaler Richtung gestrichelt dargestellt. Dies deutet an, dass der schlauchförmige Abschnitt 41 gegenüber dem Schleusengehäuse 43 innerhalb des Aufnahmekanals 46 axial verschiebbar ist oder, anders ausgedrückt, dass das Schleusengehäuse 43 auf dem schlauchförmigen Abschnitt 41 verschiebbar ist.

Die gezeigte Schleuse kann beispielsweise als Einführschleuse (erste Schleuse) eingesetzt werden.

Zum Einführen eines Funktionselementes, beispielsweise einer Pumpe, in die erste Schleuse wird üblicherweise der schlauchförmige Abschnitt 41 so weit in distaler Richtung aus dem Schleusengehäuse 43 herausgezogen oder bei der Herstellung der ersten Schleuse so positioniert, dass er etwa in Höhe des ersten Anschlagstückes 48 endet, und dann beispielsweise durch Betätigung der Druckschraube 45 festgeklemmt. Bis zu diesem Punkt kann dann eine zweite Schleuse mit einer eingezogenen Pumpe, wie oben beschrieben, vorgeschoben werden, um dann die Pumpe von der zweiten Schleuse in die erste Schleuse durchzubewegen.

Die erste Klemmvorrichtung weist als Befestigungsmittel die erste Druckschraube 45, einen ersten Klemmring 50 aus einem Elastomermaterial sowie das erste Anschlagstück 48 auf. Zwischen dem Klemmring 50 und der Druckschraube 45 kann eine Gleitschicht oder ein Gleitring 69 vorgesehen werden.

Die Druckschraube ist mittels eines Außengewindes im Bereich der Überlappung mit dem distalen Ende 44 des Schleusengehäuses 43 mit diesem verschraubt. Ein manuelles Drehen an der Druckschraube 45 bewirkt damit eine Bewegung der Druckschraube in axialer Richtung, die zu einer axialen Kompression oder Ausdehnung des Klemmrings 50 führt. Bei einer axialen Kompression hat der Klemmring 50 die Tendenz, zur Erhaltung seines Volumens radial nach innen und außen auszuweichen, und klemmt damit den schlauchförmigen Abschnitt 41, da er auf seiner proximalen Seite einen Widerstand durch das erste Anschlagstück 48 erfährt.

Damit wird der schlauchförmige Abschnitt 41 axial gegenüber dem Schleusengehäuse 43 fixiert. Diese Fixierung kann einfach durch Lösen der Druckschraube 45 aufgelöst werden, so dass der schlauchförmige Abschnitt 41 dann leicht axial in dem Schleusengehäuse 43 verschoben werden kann. Der Klemmring kann dazu im entspannten Zustand einen Innendurchmesser aufweisen, der gleich wie oder größer als der Durchmesser der ersten Schleuse ist.

Wird also zunächst der schlauchförmige Abschnitt 41 möglichst weit in den Patientenkörper hineingeschoben, um ein Einführen der Pumpe in dem Schutz der Schleuse bis zum Einsatzort, beispielsweise in einem Herzventrikel, zu ermöglichen, so wird nach dem Ausbringen der Pumpe der schlauchförmige Abschnitt 41 herausgezogen, und die Schleuse insgesamt steht relativ weit aus dem Patientenkörper vor. Danach kann die Klemmvorrichtung 48, 45, 50 gelöst und das Schleusengehäuse 43 auf dem schlauchförmigen Abschnitt 41 näher zum Patientenkörper herangeschoben werden. Dabei durchsetzt der schlauchförmige Abschnitt 41 das Schleusengehäuse 43 dann vollständig und ragt gegebenenfalls in proximaler Richtung aus diesem heraus. Darauf kann mit Mitteln, die weiter unten ausführlicher beschrieben werden, der Schlauchförmige Abschnitt 41 teilweise abgetrennt werden, um die Überlänge zu entfernen.

Innerhalb des Schleusengehäuses 43 ist zur besseren Abdichtung ein sogenanntes kombiniertes Hämostaseventil, bestehend aus einem Domventil 51 und einer Ventilplatte 52 vorgesehen. Die Ventilplatte schließt das Schleusengehäuse 43, wenn an dieser Stelle weder der schlauchförmige Abschnitt 41 noch ein Wellenkatheter den Aufnahmekanal 46 durchsetzt, während das Domventil 51 für den dichten Abschluss um einen strangförmigen Körper herum, beispielsweise den schlauchförmigen Abschnitt oder einen Katheter, optimiert ist.

Am proximalen Ende 53 des Schleusengehäuses 43 ist eine weitere Druckschraube 54 vorgesehen, die im Prinzip ebenso funktioniert wie die erste Druckschraube 45 und die über ein Druckstück 55 die Kompression eines zweiten Klemmrings 56 gegenüber einem zweiten mechanischen Anschlag 57 bewirkt. Damit kann ein Katheter oder eine eingeführte Hilfsschleuse in der Einführschleuse geklemmt werden. Als Besonderheit ist hier zu erwähnen, dass der zweite Klemmring 56 an seinem distalen Ende konisch zuläuft, was eine Verformung radial nach innen beim Ausüben eines axialen Drucks durch die Druckschraube 54 begünstigt. Entsprechend gegensinnig ist der zweite Anschlag 57 konisch ausgebildet. Es kann jedoch an dieser Stelle ebenso ein nicht konischer, sondern im Querschnitt rechteckiger oder runder Klemmring 56 eingesetzt werden.

In der Fig. 10 ist eine Spülvorrichtung 58 schematisch angedeutet, die das Spülen des Spülraums 47, der den Innenraum des Schleusengehäuses darstellt, mit einer Flüssigkeit ermöglicht, die das Eindringen von Keimen durch die erste Schleuse in den Patientenkörper verhindert. Diese Spülung ist besonders dann effektiv, wenn der schlauchförmige Abschnitt 41 in dem Spülraum 47 oder auf dessen distaler Seite endet, so dass sowohl die Außenseite als auch die Innenseite des schlauchförmigen Abschnitts 41 von der Spülflüssigkeit erreicht werden.

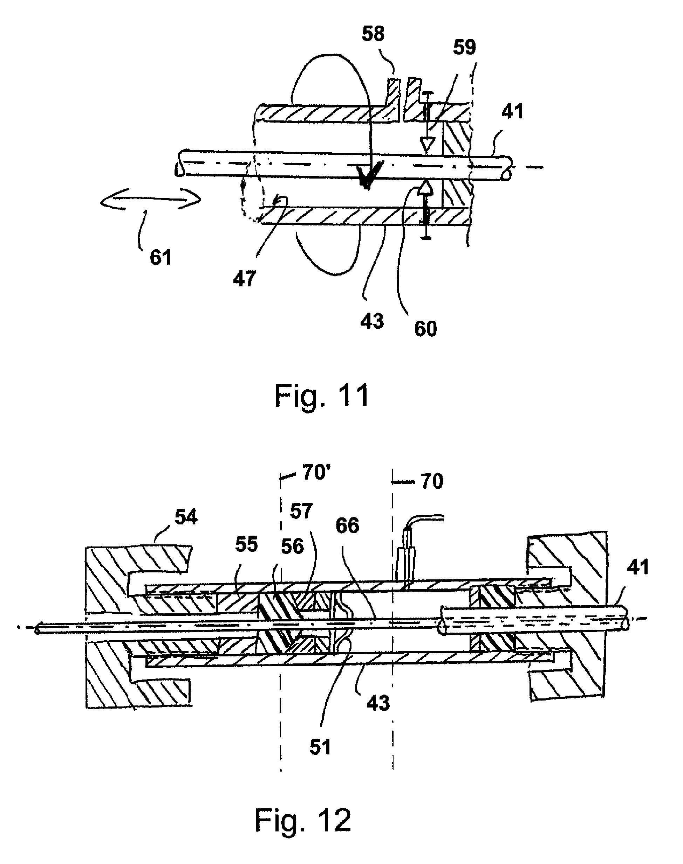

Figur 11 zeigt exemplarisch die Anordnung und Funktionsweise einer erfindungsgemäßen Schneidvorrichtung.

Wenn keine vorgeschnittenen oder in anderer Weise, beispielsweise durch eine vorgegebene Molekülstruktur oder stellenweise Schwächung der Wandstärke des schlauchförmigen Abschnitts 21, vorgegebenen Sollbruchstellen vorgesehen sind, können diese passend bei Verwendung der ersten Schleuse durch eine Schneidvorrichtung eingebracht werden. In der Fig. 11 ist im Bereich des Spülraumes 47 des Schleusengehäuses 43 eine Schneidvorrichtung mit Klingen 59, 60 vorgesehen, die beispielsweise bei einer Drehung des Schleusengehäuses gegenüber dem schlauchförmigen Abschnitt diesen in Umfangsrichtung schneiden. Es können auch Schnitte in Axialrichtung eingebracht werden.

Zu diesem Zweck können die Klingen 59, 60 auch so angeordnet sein, dass sie bei einer Bewegung des schlauchförmigen Abschnitts 41 in axialer Richtung, wie durch den Pfeil 61 angedeutet, in Längsrichtung schneiden. Es können auch sowohl Klingen für ein Schneiden in Umfangsrichtung als auch eine Klinge für ein Schneiden in Längsrichtung vorgesehen sein.

In der Fig. 11 ist außerdem dargestellt, dass die Klingen 59, 60 in Richtung radial auf den schlauchförmigen Abschnitt 41 zu durch eine Betätigung von der Außenseite des Schleusengehäuses 43 bewegt werden können. Dort können eine in Radialrichtung verlaufende Führung für einen oder mehrere Klingenhalter, eine entsprechende Dichtung und eine Federung vorgesehen sein, so dass das Eindringen von Keimen durch diese Verschiebungsvorrichtung für die Klingen verhindert wird und die Klingen im nicht betätigten Zustand radial einen Abstand zu dem schlauchförmigen Abschnitt 41 einnehmen. Nach der Verwendung der ersten Schleuse kann dann von Hand ein Druck auf die Klingen ausgeübt und der nicht benötigte Teil des schlauchförmigen Abschnitts 41 abgeschnitten werden. Ein hier nicht dargestellter Anschlag verhindert, dass die Schnitttiefe ein kritisches Maß übersteigt und dadurch ein eventuell innerhalb der Schleuse befindlicher Katheter beschädigt wird.

Die dargestellten Klingen können auch eine Schneidvorrichtung für eine zweite Schleuse bilden.

Die Figur 12 zeigt eine vorteilhafte Verwendung der zweiten Klemmvorrichtung auf der proximalen Seite des Schleusengehäuses 43, nachdem der schlauchförmige Abschnitt 41 gekürzt ist und ein Wellenkatheter 61 aus dem proximalen Ende des schlauchförmigen Abschnitts 41 heraus und weiter zu einer nicht dargestellten Kopplungsvorrichtung für eine antreibbare Welle einer Pumpe aus dem Schleusengehäuse 43 führt. Der Wellenkatheter ist in der oben angesprochenen Domdichtung 51 gedichtet, und die Klemmvorrichtung mit den Elementen der zweiten Druckschraube 54 und dem zweiten Klemmring 56, der durch das Druckstück 55 gegenüber einem zweiten Anschlag 57 axial komprimiert ist, weicht so weit radial nach innen aus, dass er den Wellenkatheter 61, der einen wesentlich geringeren Außendurchmesser aufweist als der schlauchförmige Abschnitt 41 oder eine zweite Schleuse, klemmt und insbesondere auch zusätzlich dichtet. Eine Rotation der darin drehbaren Welle wird durch die Klemmung jedoch nicht behindert. Auf diese Weise können in dem Schleusengehäuse 43 sowohl der schlauchförmige Abschnitt 41 als auch der aus diesem herausragende Wellenkatheter 61 fixiert werden.

Die zweite Klemmvorrichtung ist ebenso dazu geeignet, beim Einführen einer zweiten Schleuse in das Schleusengehäuse 43 die zweite Schleuse mit dem zweiten Klemmring 56 so zu fixieren, dass sie gegenüber dem Schleusengehäuse 43 und insbesondere auch gegenüber dem schlauchförmigen Abschnitt 41 zum Durchschieben des Wellenkatheters 61 ausreichend fixiert ist.

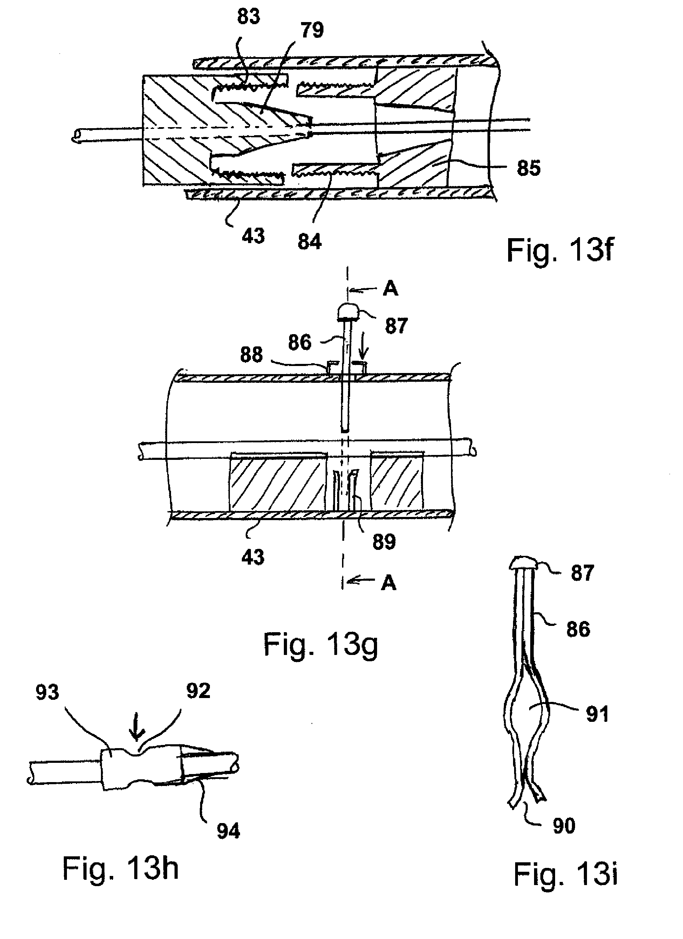

Der erste und zweite Klemmring 50, 56 können aus einem Elastomer, beispielsweise einem Gummi oder einem Silikonelastomer, bestehen und somit vollelastisch, aber volumeninkompressibel verformbar sein. Es ist an dieser Stelle jedoch auch die Verwendung eines elastischen Schaumstoffs denkbar, der teilweise volumenkompressibel ist. Anstelle eines Klemmrings 56 kann zum Herstellen einer Klemmverbindung auch eine radial verschiebbare Federklammer vorgesehen sein, wie sie in den Figuren 13g, 13h, 13i gezeigt und weiter unten erläutert ist. Eine entsprechende Federklammer kann zusätzlich zu oder anstelle von einem Klemmring vorgesehen werden. Die Federklammer kann im Bereich der gestrichelten Linien 70 in Fig. 12 vorgesehen werden. Vorteilhaft kann es jedoch auch vorgesehen sein, die Federklammer im Bereich der gestrichelten Linie 70' vorzusehen, da dieser Bereich proximal zur Dichtung 51 liegt und damit an die Dichtigkeit der Schiebeanordnung der Federklammer keine besonders hohen Anforderungen gestellt werden müssen.

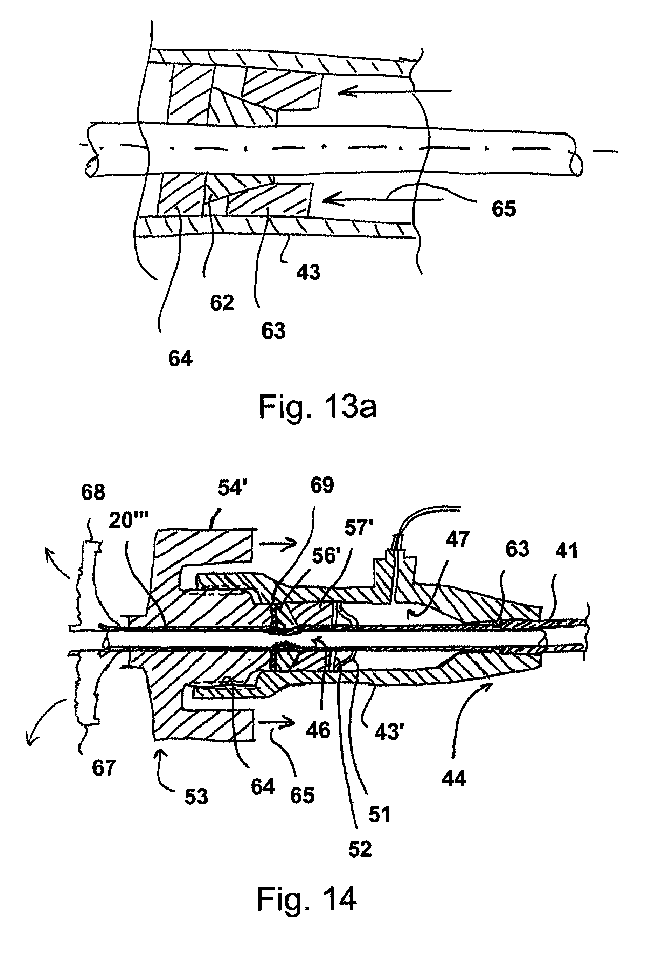

Die Figuren 13a bis 13i zeigen Klemmeinrichtungen und/oder Befestigungseinrichtungen für Schleusen, die einzeln oder in Kombination jeweils bei einer Einführschleuse, und/oder einer Hilfsschleuse eingesetzt werden können.

In der Figur 13a ist schematisch eine Art eines Klemmrings 62 gezeigt, der beispielsweise aus einem Kunststoff oder einem Metall bestehen kann und insbesondere geschlitzt und damit radial komprimierbar ist. Der geschlitzte Klemmring 62 weist eine konische Außenkontur auf, gegen die die konische Kontur eines Druckstücks 63 drückt, um den Klemmring radial zu komprimieren, sobald auf das Druckstück 63 eine axiale Druckkraft in Richtung des Pfeils 65, beispielsweise durch eine oben dargestellte Druckschraube, ausgeübt wird. Der geschlitzte Klemmring 62 ist axial durch das Anschlagstück 64 fixiert.

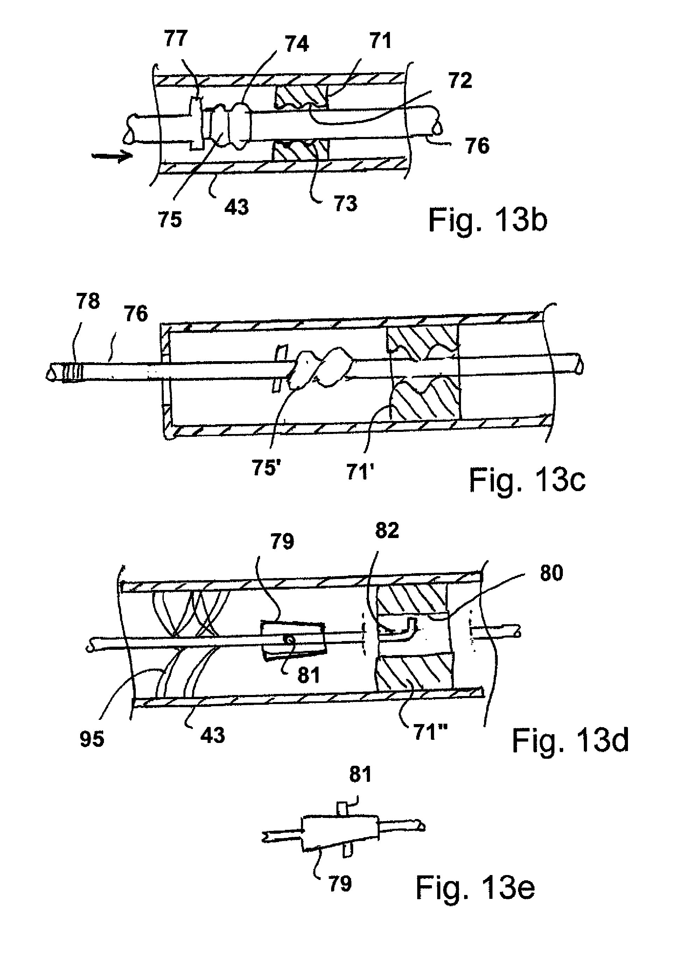

Die Figur 13b zeigt innerhalb eines Schleusengehäuses 43 ein Passstück 71, das einen Durchgangskanal 72 aufweist, wobei an der zylindrischen Mantelfläche des Durchgangskanals 72 umlaufende Rillen und Wülste 73 vorgesehen sind, die mit Rillen und Wülsten 74 eines Gegenpassstücks 75 an dem zu klemmenden Körper 76 zusammenpassen. Das Gegenpassstück 75 ist elastisch einschnappend in das Passstück 71 einschiebbar und wird dort, vorteilhaft dichtend, gehalten. Ein Anschlagstück 77 verhindert ein zu weites Einschieben des Körpers 76 und macht das Einrasten der Verbindung haptisch spürbar.

Figur 13c zeigt eine ähnliche Anordnung wie Fig. 13b, wobei das Passstück 71' und das Gegenpassstück 75' anstelle von konzentrisch umlaufenden Rillen und Wülsten jeweils mit einem Gewinde ausgestattet sind. Das Gegenpassstück 75' kann durch Drehen in das Passstück 71' bewegt werden, jedoch ist auch ein Überwinden des Gewindes und axiales Einschieben denkbar. An dem zu klemmenden Körper 76 ist zur Kennzeichnung der Zielposition eine sichtbare Marke 78 vorgesehen. Es kann jedoch auch eine Formmarkierung vorgesehen sein, die die Sollposition haptisch spürbar oder akustisch wahrnehmbar macht, oder es kann ein Mechanismus zum Betätigen eines elektrischen Kontakts zur Signalgabe bei Erreichen/Verlassen der Sollposition vorgesehen sein.

Figur 13d zeigt an dem zu klemmenden Körper 76 einen Konus 79, der in eine konische Ausnehmung 80 des Passstückes 71' dichtend hineinpasst. Der Konus 79 weist einen oder mehrere umfangsseitige Stifte 81 auf, die in Kulissen 82 an der inneren Mantelfläche der Ausnehmung 80 geführt sind und zur Ausbildung einer bajonettartigen Verriegelung dienen. Es kann auch umgekehrt am Außenkonus ein Stift und am Innenkonus eine Kulisse vorgesehen sein. Es kann bei Einhaltung der entsprechenden Standards durch die Verbindung von Dichtkonus und Verriegelung ein sogenanntes Luer-Lock gebildet sein. Anstelle der Bajonett-Verriegelung kann in Verbindung mit dem Konus-System auch eine Gewindeverbindung vorgesehen sein.

Figur 13e zeigt in einer gedrehten Seitenansicht den Konus 79 mit zwei Stiften 81.

In Figur 13f ist ein Konus-System mit einem Konus 79' und einer diesen umgebenden Gewindemutter 83 dargestellt. Die Gewindemutter 83 ist auf ein Gewinde des Rohrstutzens 84 schraubbar, der mit dem Passstück 85 verbunden ist. Das Passstück 85 ist fest in dem Schleusengehäuse 43 einer Einführschleuse eingebaut und weist einen Innenkonus auf. Die Gewindemutter 83 kann auch als frei gegenüber dem Konus 79' drehbare Überwurfmutter ausgestaltet sein.

Die Figur 13g zeigt ein Schleusengehäuse mit einer radial verschiebbar geführten Federklammer, die mittels eines Schiebeknopfes 87 betätigbar ist. Es ist ein Anschlag 88 vorgesehen, der den Schiebeknopf 87 in der Sollposition stoppt. Zudem kann an der Federklammer auch eine optisch erkennbare Markierung vorgesehen sein. Die Federklammer 86 läuft beim Einfahren in das Schleusengehäuse in eine Gegenführung 89 ein. Vorher wird die Federklammer beim Auftreffen auf den zu klemmenden Körper 76 elastisch geweitet. Hierzu weist sie, wie in Figur 13i dargestellt, eine Einlauföffnung 90 auf. Beim weiteren Einschieben legt sich der Aufnahmebereich 91 um den zu klemmenden Körper. Dieser kann, wie aus Figur 13h ersichtlich, eine umlaufende Nut oder Rille 92 aufweisen oder eine Manschette 93, die eine solche Rille 92 aufweist. Die Funktionen der Klemmung und der Dichtung an dem zu klemmenden Körper sind bei dieser Ausführungsform getrennt.

In Umkehrung der beschriebenen Funktionsweise kann die Klemmung auch dadurch hergestellt werden, dass die Federklammer voll in das Schleusengehäuse 43 eingefahren ist und erst danach der zu klemmende Körper, beispielsweise ein Katheter oder eine zweite Schleuse, axial eingeführt wird. In diesem Falle ermöglicht die konische Anlaufschräge 94, dass die Manschette 93 mit dem zu klemmenden Körper in die Federklammer 86 eingeschoben und dort gehalten werden kann.

Grundsätzlich kann der zu klemmende Körper / eine zweite Schleuse in einer ersten Schleuse auch durch elastische, selbsthemmende Lamellen, Schuppen oder Zähne gehalten werden, die bei einem Ausziehen des Körpers aus der Schleuse diesen verriegeln beziehungsweise das Herausziehen signifikant erschweren. Schematisch sind solche Lamellen auf der linken Seite der Fig. 13d dargestellt und dort mit 95 bezeichnet.

Die Fig. 14 zeigt ein Schleusengehäuse 43' einer Einführschleuse, das in seinem Inneren einen Aufnahmekanal 46 für eine Schleuse bzw. einen Katheter aufweist. Das Schleusengehäuse 43' weist an seinem distalen Ende 44 einen an diesem befestigten schlauchförmigen Abschnitt 41 auf, der beispielsweise in einer Öffnung des Schleusengehäuses eingeklebt, eingegossen oder dort anderweitig befestigt sein kann. Der schlauchförmige Abschnitt 41 ist in die Öffnung des Schleusengehäuses 43' bis zu einem mechanischen Anschlag 63 eingeschoben.

Vom proximalen Ende 53 des Schleusengehäuses 43' her ist eine zweite Schleuse 20"' (Hilfsschleuse) in den Aufnahmekanal 46 so weit eingeschoben, dass sie distal an dem mechanischen Anschlag 63 endet. In einer Ausgestaltung kann das System auch so ausgeführt werden, dass die zweite Schleuse 20'" direkt an dem schlauchförmigen Abschnitt 41 endet. In die zweite Schleuse 20"' ist, nicht mehr dargestellt, beispielsweise ein Funktionselement in Form einer Pumpe mit einem Hohlkatheter eingezogen.

Zur Überführung des Katheters mit der Pumpe von der zweiten Schleuse 20"' in den schlauchförmigen Abschnitt 41 der ersten Schleuse 43', 41 sind die beiden Schleusen innerhalb des Aufnahmekanals 46 koaxial zueinander ausgerichtet, und die zweite Schleuse 20"' ist mittels einer Klemmvorrichtung fixiert. Die Klemmvorrichtung weist einen elastischen Klemmring 56' auf, der an seinem distalen Ende konisch zulaufend ausgebildet ist und gegen einen mechanischen Anschlag 57' gedrückt wird. Zu diesem Zweck wird mittels einer Druckschraube 54', die ein Außengewinde 64 aufweist, axialer Druck auf den Klemmring 56' ausgeübt. Die Druckschraube 54' wird zu diesem Zweck in die Öffnung des röhrenförmigen Teils des Schleusengehäuses 43' eingeschraubt, so dass sie sich axial in die Richtung des Pfeils 65 bewegt.

Zur Verringerung der Reibung zwischen der sich drehenden Druckschraube 54' und dem Klemmring 56' kann vorteilhaft ein Gleitring 69, beispielsweise aus PTFE oder einem anderen gut gleitenden Kunststoff, vorgesehen sein.

Der Klemmring 56' besteht beispielsweise aus einem Elastomer, dehnt sich bei axialem Druck in radialer Richtung aus und klemmt somit einen strangförmigen Körper, der sich in dem Aufnahmekanal 46 befindet. Im Bereich der Klemmung kann der zu klemmende Körper beispielsweise eine oder mehrere umlaufende Wülste, Stege, Nuten oder Rillen oder Kanten aufweisen, um den Angriff bei der Klemmung zu verbessern. Dazu kann an dem Körper auch eine Manschette vorgesehen sein. Die zweite Schleuse 20"' weist eine Wanddicke zwischen 0,3 und 0,7 mm auf und ist aus einem ausreichend stabilen Material gefertigt, so dass bei radialem Druck die zweite Schleuse geklemmt werden kann, ohne dass gleichzeitig der darin verlaufende Katheter mit eingeklemmt wird. Der Katheter lässt sich somit leicht vom proximalen Ende der zweiten Schleuse 20'" aus in den schlauchförmigen Abschnitt 41 verschieben. Die zweite Schleuse 20"' ist durch eine kombinierte Platten- und Domdichtung 51, 52 in einem Spülraum 47 gedichtet.

Nachdem das Funktionselement, beispielsweise die Pumpe, mit dem Katheter aus der zweiten Schleuse 20'" in den schlauchförmigen Abschnitt 41 überführt worden ist, kann die zweite Schleuse anhand der Handgriffe 67, 68 aufgerissen und entfernt werden. Zu diesem Zweck kann die zweite Schleuse eine Vorschwächung oder einen Einschnitt entlang ihrer Axialrichtung oder eine entsprechend vorgegebene Molekülstruktur aufweisen, die ein Längsaufreißen bis zum distalen Ende der zweiten Schleuse und ein entsprechendes Entfernen der zweiten Schleuse ermöglicht. Zum Aufreißen kann es sinnvoll sein, die Klemmvorrichtung 54', 56', 57' zu lösen.

Nach dem Entfernen oder teilweisen Entfernen der zweiten Schleuse kann dann die Klemmvorrichtung 54', 56', 57' so weit geklemmt werden, dass der vom Durchmesser kleinere Katheter durch die weitere radiale Kompression des Klemmrings 56' in dem Aufnahmeraum 46 geklemmt wird. Damit ist der Katheter und somit auch eine zu implantierende Pumpe am distalen Ende des Katheters in Axialrichtung gegenüber der ersten Schleuse und damit gegenüber dem Patientenkörper fixiert.

Die zweite Schleuse (Hilfsschleuse) kann jedoch auch so ausgestaltet sein, dass sie in der Einführschleuse verbleiben kann. Es ist dann für eine ausreichende Abdichtung und Spülmöglichkeit zu sorgen, wie weiter unten noch erläutert wird.

Durch hier nicht dargestellte Ausformungen im Schleusengehäuse wird für die verschiedenen Endpositionen der Druckschraube, die den verschiedenen zu klemmenden Durchmessern entsprechen, jeweils ein spürbarer Anschlag realisiert, so dass der Anwender bei Erreichen der jeweiligen Klemmposition einen deutlich steigenden Drehwiderstand beim Betätigen der Schraube spürt.

Nach einer Anfangsphase, in der die Anordnung sich mechanisch setzt und auf die Körpertemperatur des Patienten erwärmt, kann die Klemmvorrichtung gelöst und der Katheter nachjustiert und darauf wieder fixiert werden. Überall, wo in der beschriebenen Konstruktion zwei zylindrische Elemente dichtend ineinandergesteckt werden, kann vorteilhaft eine Konusdichtung mit einem Konuswinkel von wenigen Grad verwendet werden, wie sie im medizinischen Bereich grundsätzlich bekannt ist.

Die beschriebene Ausführungsform einer Schleuse erlaubt es, beispielsweise eine implantierbare Herzpumpe von einer zweiten Schleuse, in der sie nach einer ersten Inspektion vorrätig gehalten werden kann, in eine erste Schleuse, die in einen Patientenkörper führt, ohne Probleme und mit geringem Aufwand sowie großer Zuverlässigkeit zu überführen,

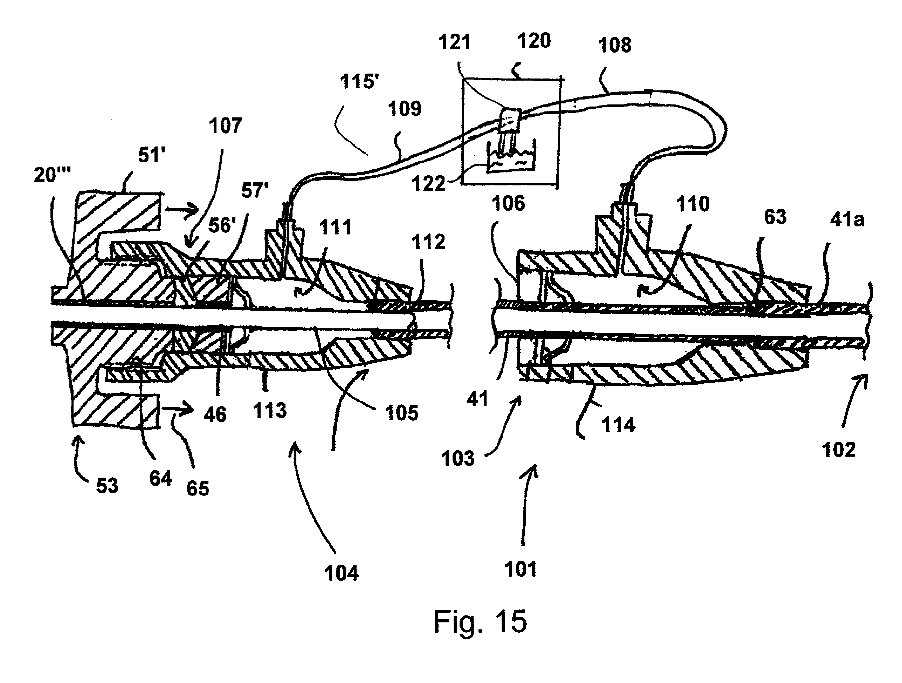

Die Figur 15 zeigt auf der rechten Seite eine Einführschleuse 101 und auf der linken Seite eine Hilfsschleuse 104, deren schlauchartiger Teil 112 in die Einführschleuse 101 einführbar und dort verklernmbar ist.

Die Einführschleuse 101 entspricht im Wesentlichen, ebenso wie die Hilfsschleuse 104, der in Fig. 14 dargestellten und in diesem Zusammenhang beschriebenen Schleuse.

Die gesamte in der Fig. 15 dargestellte Schleusenanordnung umfasst die Einführschleuse und die Hilfsschleuse und ist dazu geeignet, einen strangförmigen Körper / Katheter 105 durch die Hilfsschleuse 104 hindurch in die Einführschleuse 101 und durch diese hindurch in einen Patientenkörper einzuführen.

Die Einführschleuse 101 weist dazu an ihrem distalen Ende 102 einen schlauchförmigen Teil 41a auf, der in dem proximalen Teil 103, der ein Schleusengehäuse 114 bildet, abgedichtet befestigt ist. Der schlauchförmige Teil 112 am distalen Ende der Hilfsschleuse 104 kann in die Einführschleuse 101 eingeführt und bis zu einem Anschlag 63 vor dem schlauchförmigen Teil der Einführschleuse geschoben werden. Die Einführschleuse weist erste Befestigungsmittel 106 auf, die beispielsweise ähnlich aufgebaut sein können wie die zweiten Befestigungsmittel 107 der Hilfsschleuse 104 und die als Klemmeinrichtung näher in Verbindung mit Fig. 14 beschrieben worden sind. Grundsätzlich können die ersten und zweiten Befestigungsmittel jedoch jede der in den Figuren 13a bis 13i beispielhaft gezeigten Befestigungsarten umfassen.

Um innerhalb des Innenraums 110 des Gehäuses 114 der Einführschleuse den Übergang zwischen der Hilfsschleuse und dem schlauchartigen Teil der Einführschleuse keimfrei halten und zu diesem Zweck spülen zu können, ist eine Spüleinrichtung 108 mit einem entsprechenden Spülschlauch und einer in der Wand des Gehäuses 114 vorgesehenen Öffnung vorgesehen. Die Spüleinrichtung ist mit einer Spülmittelquelle 120 verbunden, die beispielsweise eine Pumpe 121 und einen Vorratsbehälter 122 umfasst. Die Hilfsschleuse 104 weist ebenfalls eine Spüleinrichtung 109 auf, die einen Spülschlauch und eine Öffnung in der Wand des Gehäuses 113 umfasst. Über diese Spüleinrichtung 109 kann das Gehäuseinnere 111 der Hilfsschleuse gespült und damit der Übergang von dem Katheter 105 in den schlauchartigen Teil 112 der Hilfsschleuse gespült und keimfrei gehalten werden.

Die erste Spüleinrichtung 108 ist beispielsweise mit der zweiten Spüleinrichtung 109 über die Spülmittelquelle 120 verbunden. Dabei kann vorgesehen sein, dass die Pumpe 121 sowohl in die erste Spüleinrichtung 108 als auch in die zweite Spüleinrichtung 109 Spülmittel hineinbefördert, um dies in die Innenräume der Gehäuse 113, 114 zu befördern.

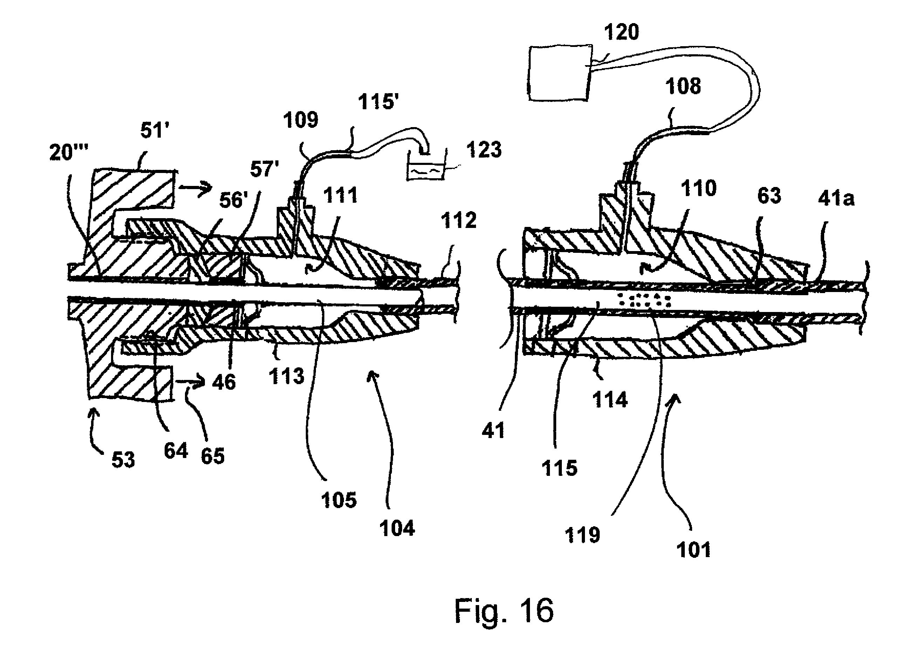

Die Figur 16 zeigt eine Variante, bei der die Spülmittelquelle 120 über die erste Spüleinrichtung 108 Spülmittel in das Gehäuseinnere 110 des Gehäuses 114 der Einführschleuse 101 liefert, wobei der innerhalb der Einführschleuse fixierte schlauchartige Teil 112 der Hilfsschleuse im Innenraum 110 wenigstens eine mantelseitige Öffnung 119, beispielsweise auch eine aus vielen Öffnungen bestehende mantelseitige Perforation, aufweist, durch die das Spülmittel in den schlauchartigen Teil 112 eindringen und durch diesen axial bis zum Gehäuseinneren 111 des Gehäuses 113 fließen kann. Damit kann aktiv das Spülmittel von der Spülmittelquelle 120 durch die Einführschleuse 101 bis in die Hilfsschleuse 104 gelangen. Die konstruktiven Einrichtungen der zweiten Spüleinrichtung 109 können in einem solchen Fall beispielsweise zur Entlüftung der Hilfsschleuse dienen oder dazu, dort die Spülflüssigkeit abzuführen, um diese in einem Sammelbehälter 123 zu sammeln. In diesem Fall stellt das Innere des schlauchartigen Teils 112 der Hilfsschleuse einen Teil des Hilfsspülkanals 115' zur Verbindung der beiden Schleusen dar. Bei der in der Fig. 15 gezeigten Variante ist ein solcher Hilfsspülkanal 115 durch die Zuleitungsschläuche der Spüleinrichtungen 108 und 109 bzw. deren Verbindung gegeben.

Die mantelseitigen Öffnungen/Ausnehmungen 119 in dem schlauchartigen Teil 112 der Hilfsschleuse können beispielsweise auch erst nach dem Einbringen des Katheters in den Patientenkörper in das Teil 112 eingebracht werden. Dazu kann beispielsweise eine Schneidenanordnung dienen, wie sie in der Fig. 11 dargestellt ist.

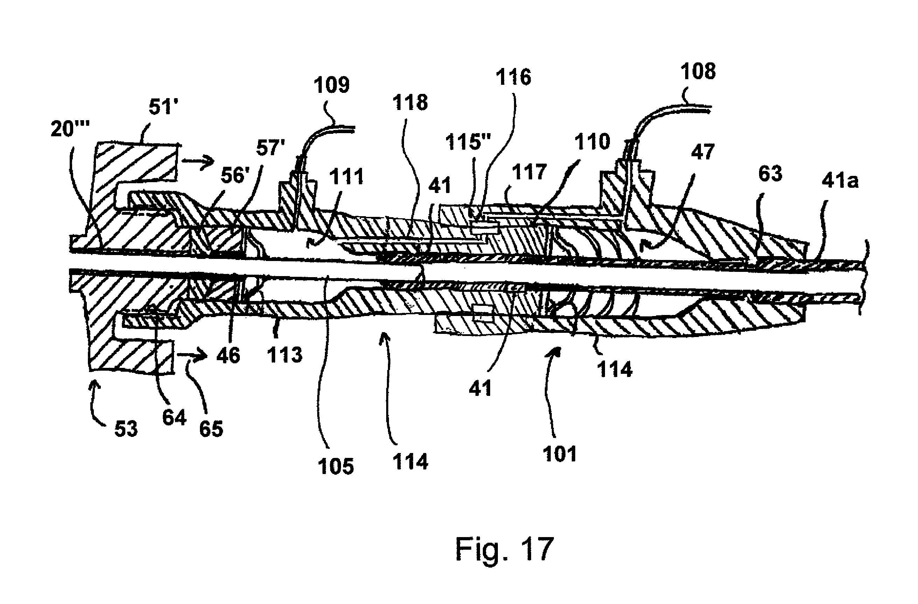

In der Figur 17 schließlich ist eine Konstruktion dargestellt, bei der das Schleusengehäuse 114 der Einführschleuse fest mit dem Schleusengehäuse 113 der Hilfsschleuse 104 verbunden ist. Die beiden Schleusengehäuse sind in einem zylindrischen oder leicht konisch geformten Teilbereich fest ineinandergeschoben. Es kann hier auch eine Gewindeverbindung oder eine Bajonettverbindung vorgesehen sein.

Es ist eine Ringnut 116 ausgebildet, die beispielsweise wie in der dargestellten Version durch Ringnuten in dem Gehäuse 113 der Hilfsschleuse einerseits und in dem Gehäuse 114 der Einführschleuse andererseits gebildet ist. Es kann aber auch ausreichen, nur in einem der Gehäuse eine Ringnut vorzusehen. Die Ringnut bzw. beide Ringnuten sind über jeweils eine kanalartige Ausnehmung 117, 118, die durch die Wand des jeweiligen Gehäuses 114, 113 verläuft, mit einer ersten Spüleinrichtung 108 bzw. dem Inneren des jeweiligen Schleusengehäuses 114, 113 verbunden. Damit kann in der gezeigten Variante über die erste Spüleinrichtung 108 einerseits das Innere des Gehäuses 114 und andererseits zusätzlich das Innere des Gehäuses 113 gespült werden. Die zweite Spüleinrichtung 109 dient in diesem Fall wie gemäß der Fig. 16 zum Entlüften und zum Ableiten von Spülflüssigkeit. Es kann aber auch umgekehrt eine Spülung beider Gehäuse durch die zweite Spüleinrichtung vorgesehen sein. Durch das Vorsehen einer Ringnut 116 muss keine genaue Winkelposition bei der Verbindung der beiden Schleusengehäuse beachtet werden, sondern lediglich die axiale Position der beiden Schleusengehäuse 101, 104 zueinander muss eingehalten werden. Dies kann beispielsweise durch einen festen Anschlag an einem der beiden Schleusengehäuse sichergestellt werden.

Es kann zudem jeweils durch einen federnden Schieber an der inneren Umfangsseite des Gehäuses 114 und an der Außenseite des Gehäuses 113 jeweils im Bereich der Ringnut sichergestellt werden, dass die entsprechenden Ringnuten und/oder kanalartigen Ausnehmungen vor der Verbindung der beiden Gehäuse abgedeckt sind. Die Schieber sollten federbelastet und derart zurückschiebbar sein, dass sie beim Zusammenfügen der beiden Gehäuse die jeweiligen Ringnuten oder Ausnehmungen freigeben.

Die kanalartigen Ausnehmungen 117, 118 bzw. die Ringnut 116 bilden im Falle der Konstruktion aus Fig. 17 den Hilfsspülkanal 115".

Durch die beschriebenen Varianten wird es möglich, die Hilfsschleuse 104 dauerhaft an der Einführschleuse, 101 und mit dieser verbunden zu belassen und beide gemeinsam derart zu spülen, dass im Bereich der Schleuse das Eindringen von Keimen in den Patientenkörper zu verlässig verhindert werden kann.

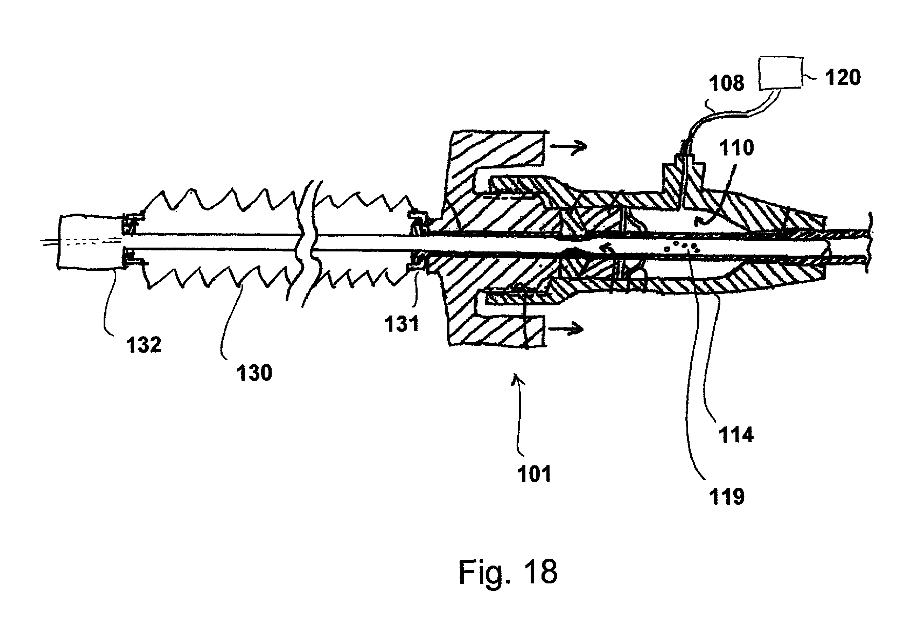

Die Figur 18 zeigt eine Gestaltung der Schleusenanordnung, bei der an einer Einführschleuse 101 ein Sterilschlauch 130 fluiddicht befestigt ist. Dieser kann grundsätzlich von der Einführschleuse 101 oder einer Hilfsschleuse 104' (in Fig. 18 nur teilweise und schematisch dargestellt) aus spülbar sein.

Der Sterilschlauch 130 ist selbst fluiddicht und als Wellschlauch ausgebildet. Er kann mittels einer Flansch- oder Klebeverbindung 131 oder allgemein mittels einer der in den Figuren 13a bis 13i gezeigten Verbindungsarten mit dem Gehäuse der Einführschleuse 114 und/oder dem Gehäuse der Hilfsschleuse und/oder einem Kathetergriff verbunden sein und umgibt wenigstens teilweise eine Hilfsschleuse, die teilweise in die Einführschleuse eingebracht ist. Der Sterilschlauch kann proximal auch direkt an den Katheterschaft geklemmt werden/sein.

Nach dem Einbringen eines Katheters in einen Patientenkörper kann die Hilfsschleuse vollständig in den Sterilschlauch 130 aufgenommen werden. Ein proximales Endstück 132 weist einen Durchlasskanal für den Katheter auf, der dort dichtend durchgeführt ist.

Nach dem Einführvorgang kann der Sterilschlauch axial komprimiert werden, und es kann beispielsweise das Endstück 132 mit einem Flansch 131 verbunden werden. Es kann auch die Hilfsschleuse als Peel-away-Schleuse aufgerissen und aus der Einführschleuse in den Sterilschlauch hineingezogen werden, um dort zu verbleiben. Die Einführschleuse kann dann gegenüber dem Sterilschlauch abgedichtet sein.

## Patentansprüche

1. Schleusenanordnung für die Einführung eines strangförmigen Körpers (32, 105), insbesondere eines Katheters, in einen Patientenkörper mit einer Einführschleuse (101), deren distales Ende (102) zum Einbringen in einen Patientenkörper vorgesehen ist und deren proximales Ende (103) beim Einbringen des distalen Endes in einen Patientenkörper aus diesem herausragt, und mit einer Hilfsschleuse (104)zur Einführung in die Einführschleuse (101) gemeinsam mit dem strangförmigen Körper / Katheter (32, 105), mit ersten Befestigungsmitteln (106) zur, insbesondere lösbaren, Befestigung der Hilfsschleuse an der Einführschleuse, mit zweiten Befestigungsmitteln (107) zur, insbesondere lösbaren, Befestigung des strangförmigen Körpers an der Hilfsschleuse, wobei die Einführschleuse (101) einen ersten Schleuseninnenraum (110) mit einem ersten Aufnahmekanal für einen strangförmigen Körper (32, 105) sowie eine erste Spüleinrichtung (108) zum Spülen des Schleuseninnenraums (110) aufweist, wobei die Hilfsschleuse (104) einen zweiten Schleuseninnenraum (111) mit einem zweiten Aufnahmekanal für einen strangförmigen Körper (32, 105)sowie eine zweite Spüleinrichtung (109) für den zweiten Schleuseninnenraum (111) aufweist.

2. Schleusenanordnung nach Anspruch 1, **dadurch gekennzeichnet, dass** die Hilfsschleuse (104) einen schlauchartigen, distalen Teil (112) und ein proximales Schleusengehäuse (113) aufweist und dass nur der schlauchartige Teil (112) mit der Einführschleuse (101), insbesondere lösbar, verbunden ist.

3. Schleusenanordnung nach Anspruch 1, **dadurch gekennzeichnet, dass** die Hilfsschleuse (104) einen schlauchartigen, distalen Teil (112) und ein Schleusengehäuse (113) aufweist und dass das Schleusengehäuse (113) der Hilfsschleuse, insbesondere lösbar, mit der Einführschleuse (101), insbesondere mit einem Schleusengehäuse (114) der Einführschleuse (101), verbunden ist.

4. Schleusenanordnung nach einem der Ansprüche 1 bis 3, **dadurch gekennzeichnet, dass** die Verbindung der Hilfsschleuse (104) mit der Einführschleuse (101) als lösbare Klemmverbindung mit Kraft- oder Reibschluss, als Doppelkonusverbindung, Schraubverbindung, Bajonettverbindung, Presspassung oder andere lösbare Passverbindung ausgeführt ist.

5. Schleusenanordnung nach einem der Ansprüche 1 bis 4, **dadurch gekennzeichnet, dass** die zweite Spüleinrichtung (109) mit der ersten Spüleinrichtung (108) zum Austausch von Spülfluid verbunden ist.

6. Schleusenanordnung nach Anspruch 5, **dadurch gekennzeichnet, dass** der Innenraum des Schleusengehäuses (113) der Hilfsschleuse mittels eines Hilfsspülkanals (115, 115', 115") unmittelbar mit der ersten Spüleinrichtung (108) verbunden ist.

7. Schleusenanordnung nach Anspruch 6, **dadurch gekennzeichnet, dass** der Hilfsspülkanal (115, 115', 115") durch eine umfangsseitig außen am Gehäuse der Hilfsschleuse (104) angeschlossene Leitung in Form eines Schlauchs oder Rohrs verläuft.

8. Schleusenanordnung nach Anspruch 6, **dadurch gekennzeichnet, dass** der Hilfsspülkanal (115, 115', 115") unmittelbar vom Schleusengehäuse (114) der Einführschleuse (101) in das Innere des schlauchartigen Teils (112) der Hilfsschleuse (104) verläuft, insbesondere durch mantelseitige Öffnungen (119) in dem schlauchartigen Teil (112) der Hilfsschleuse.

9. Schleusenanordnung nach Anspruch 6, **dadurch gekennzeichnet, dass** im Fall einer unmittelbaren Verbindung des Schleusengehäuses (113) der Hilfsschleuse mit dem Schleusengehäuse (114) der Einfuhrschleuse der Hilfsspülkanal (115, 115', 115") innerhalb des Verbindungsbereichs, insbesondere durch einen innerhalb der Einführschleuse (101) angeordneten Bereich der Wand des Schleusengehäuses (113) der Hilfsschleuse hindurch, verläuft.

10. Schleusengehäuse nach Anspruch 9, **dadurch gekennzeichnet, dass** die Einführschleuse (101) und die Hilfsschleuse (104) in einem ringförmigen Bereich als Verbindungspartner aneinander anliegen und dass in dem ringförmigen Bereich eine ringförmig oder ringsegmentförmig in Umfangsrichtung umlaufende Ringnut (116) in wenigstens einem der beiden Verbindungspartner vorgesehen ist und dass sowohl in dem Schleusengehäuse (114) der Einführschleuse als in dem Schleusengehäuse (113) der Hilfsschleuse jeweils eine kanalartige Ausnehmung (117, 118) vorgesehen ist, die den Schleuseninnenraum der jeweiligen Schleuse im verbundenen Zustand mit der Ringnut (116) verbindet.

11. Schleusenanordnung nach einem der vorangehenden Patentansprüche, **dadurch gekennzeichnet, dass** mit der Einführschleuse (101) ein Sterilschlauch (130) fluiddicht verbunden ist, der eine proximale Verlängerung der Einführschleuse (101) bildet und wenigstens Teile einer Hilfsschleuse (104) umgibt, wobei der Sterilschlauch mit der Hilfsschleuse (104) fluiddicht verbunden ist.
